# EUROPEAN PATENT SPECIFICATION

(11) **EP 4 161 447 B1**
(45) Date of publication and mention of the grant of the patent: **27.05.2026**
(21) Application number: 21752800.9
(22) Date of filing: 13.07.2021
(51) Int. Cl.: A61F 2/24

(54) **CLAMPS FOR COUPLING EXPANSION AND LOCKING ASSEMBLIES TO FRAMES OF PROSTHETIC VALVES**
KLEMMEN ZUR KOPPLUNG VON EXPANSIONS- UND VERRIEGELUNGSANORDNUNGEN AN RAHMEN VON HERZKLAPPENPROTHESEN
PINCES D'ACCOUPLEMENT D'ENSEMBLES DE DÉPLOIEMENT ET DE VERROUILLAGE À DES ARMATURES DE VALVULES PROTHÉTIQUES

(30) Priority: 13.07.2020 US 202063050934 P
(43) Date of publication of application: 12.04.2023
(73) Proprietor: Edwards Lifesciences Corporation, Irvine, CA 92614-5688 (US)
(72) Inventor: MAIMON, David, 30889 Caesarea (IL)
(74) Representative: Eisenführ Speiser
(86) International application number: PCT/US2021/041361
(87) International publication number: WO 2022/015683

(56) References cited:
- WO-A1-2020/081893
- WO-A1-2020/102487
- US-A1- 2018 153 689

## Description

### FIELD OF THE INVENTION

The present disclosure relates to implantable, mechanically expandable prosthetic devices, such as prosthetic heart valves, and to devices and methods for coupling expansion and locking assemblies to frames of such prosthetic devices.

### BACKGROUND OF THE INVENTION

Native heart valves, such as the aortic, pulmonary and mitral valves, function to assure adequate directional flow from and to the heart, and between the heart's chambers, to supply blood to the whole cardiovascular system. Various valvular diseases can render the valves ineffective and require replacement with artificial valves. Surgical procedures can be performed to repair or replace a heart valve. Surgeries are prone to an abundance of clinical complications, hence alternative less invasive techniques of delivering a prosthetic heart valve over a catheter and implanting it over the native malfunctioning valve, have been developed over the years.

Mechanically expandable valves are a category of prosthetic valves that rely on a mechanical actuation mechanism for expansion. The actuation mechanism usually includes a plurality of actuation/locking assemblies, releasably connected to respective actuation members of the valve delivery system, controlled via the handle for actuating the assemblies to expand the valve to a desired diameter. The assemblies may optionally lock the valve's position to prevent undesired recompression thereof, and disconnection of the delivery system's actuation member from the valve actuation/locking assemblies, to enable retrieval thereof once the valve is properly positioned at the desired site of implantation.

Despite the recent advancements in prosthetic valve technology, there remains a need for improved transcatheter heart valves and delivery systems for such valves.

WO 2020/102487 Al relates to a prosthetic heart valve which includes an annular frame including a plurality of angled strut members that is radially collapsible to a collapsed configuration and radially expandable to an expanded configuration. The frame has an inflow end and an outflow end, and includes a leaflet structure positioned within the frame, the leaflet structure comprising a plurality of leaflets arranged to form a plurality of commissures. The frame includes a plurality of commissure support elements, each commissure support element being positioned at one of the commissures. Each of the commissure support elements has a coupling portion coupled to the frame and first and second members coupled to the coupling portion and extending in a direction toward the inflow end of the frame or toward the outflow end of the frame. The leaflets of each commissure are received between the first and second members of the respective commissure support element.

### SUMMARY OF THE DISCLOSURE

The present invention is defined in the appended claims. The present disclosure is directed toward devices and assemblies for expanding and locking prosthetic valves, as well as related methods and devices for such assemblies. In several embodiments, the disclosed assemblies are configured for delivering replacement heart valves into a heart of a patient, wherein the replacement heart valves may be expanded and locked in a desired diameter at the implantation site.

According to an aspect of the disclosure, there is provided a prosthetic valve comprising a frame movable between a radially compressed and a radially expanded configuration, at least one clamp coupled to the frame, and at least one expansion and locking mechanism. The at least one clamp comprises a clamp-mid portion and a couple of opposing side arms, continuously extending from the clamp mid-portion. The at least one expansion and locking mechanism comprises an outer member and an inner member. The outer member is coupled to the frame at a first location. The inner member is coupled to the frame at a second location spaced apart from the first location, the inner member extending at least partially into the outer member.

The movement of the inner member in a first direction, relative to the outer member, causes the frame to foreshorten axially and expand radially. The side arms are resiliently expandable from each other, and are inwardly biased toward each other in the absence of an expanding force applied thereto. At least one of: the outer member and/or the inner member, is coupled to the frame via the at least one clamp.

According to some embodiments, the at least one clamp further comprises an opening extending through the thickness of the clamp mid-portion.

According to some embodiments, the at least one clamp is coupled o the frame via a fastener extending through the opening.

According to some embodiments, the at least one clamp further comprises a clamp fastening extension, extending radially outward from the clamp mid-portion.

According to some embodiments, the frame comprises a plurality of inner struts and a plurality of outer struts, pivotably interconnected at junctions, and wherein the at least one clamp is attached to an inner strut and an outer strut at a junction formed therebetween.

According to some embodiments, the at least one clamp is disposed radially inward with respect to the inner strut.

According to some embodiments, the at least one clamp is disposed between the inner strut and the outer strut.

According to some embodiments, each of the side arms comprises an offsetting portion continuously extending from the clamp mid-portion, and an arcuate portion continuously extending from the offsetting portion.

According to some embodiments, the at least one clamp further comprises clamp axial arms, extending axially from the side arms.

According to some embodiments, the clamp axial arms are oriented in a proximal direction.

According to some embodiments, the clamp axial arms are offset radially inward with respect to the outer member.

According to some embodiments, the clamp further comprises slits extending through the thickness of the side arms.

According to some embodiments, the clamp is C-shaped.

According to some embodiments, the outer member further comprises an outer member coupling recess, configured to accommodate at least a portion of the at least one clamp.

According to some embodiments, the depth of the outer member coupling recess is equal to, or greater than, the thickness of the at least one clamp.

According to some embodiments, the inner member further comprises an inner member coupling recess, configured to accommodate at least a portion of the at least one clamp.

According to some embodiments, the depth of the inner member coupling recess is equal to, or greater than, the thickness of the at least one clamp.

According to some embodiments, the expansion and locking assembly further comprises a distal nut configured to engage with the inner member, and wherein the distal nut comprises a nut coupling recess, configured to accommodate at least a portion of the at least one clamp.

According to some embodiments, the depth of the nut coupling recess is equal to, or greater than, the thickness of the at least one clamp.

According to some embodiments, the at least one clamp comprises a first clamp coupled to the frame at the first location, and a second clamp coupled to the frame at the second location, wherein the outer member is coupled to the first clamp, and wherein the inner member is coupled to the second clamp.

According to another aspect of the invention, there is provided a prosthetic valve comprising a frame movable between a radially compressed and a radially expanded configuration, at least one clamp coupled to the frame, and at least one expansion and locking mechanism. The at least one clamp comprises a clamp-mid portion comprising an opening extending through its thickness, and a couple of opposing side arms, continuously extending from the clamp mid-portion. The at least one expansion and locking mechanism comprises an outer member and an inner member. The outer member is coupled to the frame at a first location. The inner member is coupled to the frame at a second location spaced apart from the first location, the inner member extending at least partially into the outer member.

The movement of the inner member in a first direction, relative to the outer member, causes the frame to foreshorten axially and expand radially. The side arms are resiliently expandable from each other, and are inwardly biased toward each other in the absence of an expanding force applied thereto. At least one of: the outer member and/or the inner member, is coupled to the frame via the at least one clamp.

According to some embodiments, the frame comprises a plurality of inner struts and a plurality of outer struts, pivotably interconnected at junctions, wherein the at least one clamp is attached to an inner strut and an outer strut at a junction formed therebetween.

According to some embodiments, the clamp mid-portion is disposed radially inward with respect to the frame.

According to some embodiments, the clamp mid-portion is disposed between the inner strut and the outer strut.

According to some embodiments, the opening of the clamp is aligned with an aperture of the inner strut and an aperture of the outer strut.

According to some embodiments, the at least one clamp is coupled to the frame via a fastener extending through the opening, the aperture of the inner strut, and the aperture of the outer strut.

According to some embodiments, the fastener comprises a fastener main body and an enlarged fastener head.

According to some embodiments, the aperture of the outer strut is dimensioned to accommodate a portion of the fastener main body, wherein the aperture of the inner strut comprises a main bore configured to accommodate a portion of the fastener main body, and a wider counter-bore configured to accommodate the fastener head.

According to some embodiments, the the inner strut comprises an integral fastener, wherein the inner strut, the at least one clamp, and the outer strut are coupled together via the integral fastener extending through the opening and an aperture of the outer strut.

According to some embodiments, the outer member further comprises an outer member fastening extension, wherein the at least one clamp is coupled to the frame at the first location via the outer member fastening extension, extending through the opening, the aperture of the inner strut, and the aperture of the outer strut.

According to some embodiments, the inner member further comprises an inner member fastening extension, wherein the at least one clamp is coupled to the frame at the second location via the inner member fastening extension, extending through the opening, the aperture of the inner strut, and the aperture of the outer strut.

According to some embodiments, each of the side arms comprises an offsetting portion continuously extending from the clamp mid-portion, and an arcuate portion continuously extending from the offsetting portion.

According to some embodiments, the at least one clamp further comprises clamp axial arms, extending axially from the side arms.

According to some embodiments, the clamp axial arms are oriented in a proximal direction.

According to some embodiments, the clamp axial arms are offset radially inward with respect to the outer member.

According to some embodiments, the prosthetic valve further comprises a plurality of leaflets, wherein the tabs of each two adjacent leaflets are coupled to the clamp axial arms, thereby forming a commissure.

According to some embodiments, the clamp further comprises slits extending through the thickness of the side arms.

According to some embodiments, the clamp is C-shaped.

According to some embodiments, the outer member further comprises an outer member coupling recess, configured to accommodate at least a portion of the at least one clamp.

According to some embodiments, the outer member coupling recess has a depth which is equal to, or greater than, the thickness of the at least one clamp.

According to some embodiments, the outer member coupling recess has a height which is not greater than 110% of the longitudinal length of the at least one clamp.

According to some embodiments, the inner member further comprises an inner member coupling recess, configured to accommodate at least a portion of the at least one clamp.

According to some embodiments, the inner member coupling recess has a depth which is equal to, or greater than, the thickness of the at least one clamp.

According to some embodiments, the inner member coupling recess has a height which is not greater than 110% of the longitudinal length of the at least one clamp.

According to some embodiments, a gap defined between free ends of the side arms at a free state of the at least one clamp, is smaller than a width of the outer member at the region of the outer member configured to snap into the at least one clamp.

According to some embodiments, a gap defined between free ends of the side arms at a free state of the at least one clamp, is smaller than a width of the inner member at the region of the inner member configured to snap into the at least one clamp.

According to some embodiments, the at least one expansion and locking assembly further comprises a distal nut configured to engage with the inner member, and wherein the distal nut comprises a nut coupling recess, configured to accommodate at least a portion of the at least one clamp.

According to some embodiments, the nut coupling recess has a depth which is equal to, or greater than, the thickness of the at least one clamp.

According to some embodiments, a gap defined between free ends of the side arms at a free state of the at least one clamp, is smaller than a width of the distal nut at the region of the distal nut configured to snap into the at least one clamp.

According to some embodiments, the at least one clamp comprises a first clamp coupled to the frame at the first location, and a second clamp coupled to the frame at the second location, wherein the outer member is coupled to the first clamp, and wherein the inner member is coupled to the second clamp.

According to yet another aspect of the invention, there is provided a prosthetic valve comprising a frame movable between a radially compressed and a radially expanded configuration, at least one clamp coupled to the frame, and at least one expansion and locking mechanism. The at least one clamp comprises a clamp-mid portion comprising a clamp fastening extension extending radially outward therefrom, and a couple of opposing side arms, continuously extending from the clamp mid-portion. The at least one expansion and locking mechanism comprises an outer member and an inner member. The outer member is coupled to the frame at a first location. The inner member is coupled to the frame at a second location spaced apart from the first location, the inner member extending at least partially into the outer member.

The movement of the inner member in a first direction, relative to the outer member, causes the frame to foreshorten axially and expand radially. The side arms are resiliently expandable from each other, and are inwardly biased toward each other in the absence of an expanding force applied thereto. At least one of: the outer member and/or the inner member, is coupled to the frame via the at least one clamp.

According to some embodiments, the frame comprises a plurality of inner struts and a plurality of outer struts, pivotably interconnected at junctions, and wherein the at least one clamp is attached to an inner strut and an outer strut at a junction formed therebetween.

According to some embodiments, the clamp mid-portion is disposed radially inward with respect to the frame.

According to some embodiments, the at least one clamp is coupled to the frame via the clamp fastening extension, extending through an aperture of the inner strut and an aperture of the outer strut.

According to some embodiments, the aperture of the outer strut and the aperture of the inner strut are each dimensioned to accommodate a portion of the clamp fastening extension.

According to some embodiments, each of the side arms comprises an offsetting portion continuously extending from the clamp mid-portion, and an arcuate portion continuously extending from the offsetting portion.

According to some embodiments, the at least one clamp further comprises clamp axial arms, extending axially from the side arms.

According to some embodiments, the clamp axial arms are oriented in a proximal direction.

According to some embodiments, the clamp axial arms are offset radially inward with respect to the outer member.

According to some embodiments, the prosthetic valve further comprises a plurality of leaflets, wherein the tabs of each two adjacent leaflets are coupled to the clamp axial arms, thereby forming a commissure.

According to some embodiments, the clamp further comprises slits extending through the thickness of the side arms.

According to some embodiments, the clamp is C-shaped.

According to some embodiments, the outer member further comprises an outer member coupling recess, configured to accommodate at least a portion of the at least one clamp.

According to some embodiments, the outer member coupling recess has a has a depth which is equal to, or greater than, the thickness of the at least one clamp.

According to some embodiments, the outer member coupling recess has a height which is not greater than 110% of the longitudinal length of the at least one clamp.

According to some embodiments, the inner member further comprises an inner member coupling recess, configured to accommodate at least a portion of the at least one clamp.

According to some embodiments, the inner member coupling recess has a depth which is equal to, or greater than, the thickness of the at least one clamp.

According to some embodiments, the inner member coupling recess has a height which is not greater than 110% of the longitudinal length of the at least one clamp.

According to some embodiments, a gap defined between free ends of the side arms at a free state of the at least one clamp, is smaller than a width of the outer member at the region of the outer member configured to snap into the at least one clamp.

According to some embodiments, a gap defined between free ends of the side arms at a free state of the at least one clamp, is smaller than a width of the inner member at the region of the inner member configured to snap into the at least one clamp.

According to some embodiments, the at least one expansion and locking assembly further comprises a distal nut configured to engage with the inner member, and wherein the distal nut comprises a nut coupling recess, configured to accommodate at least a portion of the at least one clamp.

According to some embodiments, the nut coupling recess has a depth which is equal to, or greater than, the thickness of the at least one clamp.

According to some embodiments, the at least one clamp comprises a first clamp coupled to the frame at the first locations, and a second clamp coupled to the frame at the second location, wherein the outer member is coupled to the first clamp, and wherein the inner member is coupled to the second clamp.

According to yet another aspect of the invention, there is provided a method of assembling a prosthetic valve, the method comprises providing a frame that includes a plurality of inner struts and outer struts, pivotably coupled to each other at junctions thereof.

The method further comprises coupling at least one clamp to a junction of the frame, wherein the at least one clamp comprises a clamp mid-portion, and a couple of opposing side arms continuously extending from the clamp mid-portion.

The method further comprises coupling a component of an expansion and locking assembly to the at least one clamp by sliding the component through the resilient side arms, thereby expanding them away from each other, and allowing the side arms to snap back toward each other once the component is situated within the clamp. The component of the expansion and locking assembly comprises an outer member and/or an inner member, configured to expand the prosthetic valve when moved axially toward each other

According to some embodiments, the clamp mid-portion comprises an opening extending through its thickness.

According to some embodiments, coupling at least one clamp to a junction includes placing the clamp mid-portion radially inward to the frame or between the inner strut and the outer strut, such that the opening is aligned with an aperture of the inner strut and an aperture of the outer strut.

According to some embodiments, coupling the at least one clamp to a junction further includes inserting a fastener through the opening, and through the apertures of the inner and the outer struts

According to some embodiments, coupling at least one clamp to a junction includes placing the clamp mid-portion between the inner strut and the outer strut, and extending an integral fastener of the inner strut through the opening and through an aperture of the outer strut.

According to some embodiments, coupling the at least one clamp to a junction further includes extending an outer member fastening extension of an outer member, through the opening, and through the apertures of the inner and the outer struts, at a first location.

According to some embodiments, coupling the at least one clamp to a junction further includes extending an inner member fastening extension of an inner member, through the opening, and through the apertures of the inner and the outer struts, at a second location.

According to some embodiments, the clamp mid-portion comprises a clamp fastening extension.

According to some embodiments, coupling at least one clamp to a junction includes placing the clamp mid-portion radially inward to the frame, and extending the clamp fastening extension through an aperture of the inner strut and an aperture of the outer strut.

According to some embodiments, coupling the component of an expansion and locking assembly to the at least one clamp includes snaping the side arms over a recess of the component.

According to some embodiments, the at least one clamp comprises a first clamp having axial arms extending axially from the side arms, wherein coupling the component of an expansion and locking assembly to the at least one clamp comprises coupling the outer member to the first clamp.

According to some embodiments, coupling the outer member to the first clamp is performed such that the clamp axial arms are extending proximally from the side arms.

According to some embodiments, the method further comprises attaching commissures to the prosthetic valves, by coupling leaflet tabs of each couple of adjacent leaflets of a commissure to the clamp axial arms.

The various innovations of this disclosure can be used in combination or separately. This summary is provided to introduce a selection of concepts in a simplified form that are further described below in the detailed description.

The foregoing and other objects, features, and advantages of the invention will become more apparent from the following detailed description, which proceeds with reference to the accompanying figures.

### BRIEF DESCRIPTION OF THE FIGURES

Some embodiments of the invention are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the invention. For the sake of clarity, some objects depicted in the figures are not to scale.

In the Figures:
Fig. 1 is a view in perspective of a prosthetic valve, according to some embodiments.
Fig. 2A is a view in perspective of an inner member, according to some embodiments.
Fig. 2B is a view in perspective of an expansion and locking assembly, according to some embodiments.
Fig. 2C is a view in perspective of a prosthetic valve including multiple expansion and locking assemblies of the type shown in Fig. 2B.
Figs. 3A-3C show an expansion and locking assembly of the type shown in Fig. 2B in different operational states thereof.
FIG. 4 is a plan view of a strut of the frame of FIG. 1, shown in a flattened configuration.
Fig. 5 is a partial view in perspective of an outflow apex of the frame of FIG. 1, according to some embodiments.
FIG. 6 shows an enlarged cross-sectional view of the connection between two struts via a fastener, according to some embodiments.
Fig. 7A is a partial view in perspective of a strut provided with uniformly shaped apertures, according to some embodiments.
Fig. 7B is a partial view in perspective of a strut provided with apertures having counter-bores, according to some embodiments.
FIG. 8 shows an enlarged cross-sectional view of the connection between two frame struts of the types shown in Figs. 7A and 7B, according to some embodiments.
Fig. 9 is a view in perspective of a strut provided with integral fasteners, according to some embodiments.
Figs. 10A-10B are cross-sectional views of the connection between the strut of Fig. 9 with the strut of Fig. 7A, shown before and after engagement therebetween, respectively.
Fig. 11A is a view in perspective of a strut provided with a plurality of integral fasteners and an aperture extending through a strut end portion, according to some embodiments.
Fig. 11B is a view in perspective of a strut provided with a plurality of integral fasteners and an aperture extending through an intermediate segment, according to some embodiments.
FIG. 12 is a partial sectional view of an expansion and locking coupled to the frame via fastening extension of the assembly, according to some embodiments.
Fig. 13A is a view in perspective of a clamp provided with arching side arms and an opening extending through the clamp mid-portion, according to some embodiments.
Fig. 13B is a cross-sectional view of the clamp shown in Fig. 13A.
Fig. 14 is a view in perspective of an outer member having a coupling recess, according to some embodiments.
Fig. 15 is a view in perspective of the clamp of Fig. 13A coupled to the frame, according to some embodiments.
Figs. 16A-16B are cross-sectional views the clamp of Fig. 13A disposed radially inward with respect to two struts, and coupled together via a fastener, shown before and after engagement therebetween, respectively.
Fig. 17 is a view in perspective of an outer member coupled to the frame via a clamp, according to some embodiments.
Fig. 18 is a view in perspective of a clamp provided offsetting portions and arcuate portions of the side arms, according to some embodiments.
Fig. 19A is a view in perspective of the clamp of Fig. 18 coupled to the frame between an inner strut and an outer strut, according to some embodiments.
Fig. 19B is a cross-sectional view of an expansion and locking assembly coupled to the frame via the clamp of Fig. 18, according to some embodiments.
Figs. 20A-20B are cross-sectional views the clamp of Fig. 18 disposed between two struts, and coupled together via a fastener, shown before and after engagement therebetween, respectively.
Figs. 21A-21B are back and front views in perspective of an outer member, provided with a fastening extension extending radially outward from its coupling recess, according to some embodiments.
FIG. 22 is a partial sectional view of an outer member coupled to the frame via fastening extension extending through a clamp, according to some embodiments.
Fig. 23A is a view in perspective of a clamp provided with a clamp fastening extension, according to some embodiments.
Fig. 23B is a cross-sectional view of the clamp shown in Fig. 23A.
Figs. 24A-24B are cross-sectional views the clamp of Fig. 23A attached to a couple of struts, shown before and after engagement therebetween, respectively.
Fig. 25A is a view in perspective of an inner member provided with a coupling recesses spanning over a portion of a widened distal end portion thereof, according to some embodiments.
Fig. 25B is a view in perspective of an inner member provided with a coupling recesses spanning over a portion of a circular end portion thereof, according to some embodiments.
FIG. 26 is a sectional view of an expansion and locking assembly coupled to the frame via a first clamp coupled to the outer member, and a second clamp coupled to the inner member, according to some embodiments.
Fig. 27A is a view in perspective of a clamp provided with clamp axial arms, according to some embodiments.
Fig. 27B is a view in perspective of a clamp provided with clamp axial arms and with slits, according to some embodiments.
Fig. 27C is a view in perspective of a clamp provided with clamp axial arms and with a clamp fastening extension, according to some embodiments.
Fig. 28A is a view in perspective of a C-shaped clamp, according to some embodiments.
Fig. 28B is a cross-sectional view of the clamp shown in Fig. 28A.
Fig. 29A is a view in perspective of a screw-shaped inner member, according to some embodiments.
Fig. 29B is an expansion and locking assembly comprising the screw-shaped inner member of Fig. 29A, according to some embodiments.
Fig. 30A is a view in perspective of the expansion and locking mechanism of Fig. 29B, wherein the outer member is coupled to the frame via the clamp of Fig. 27A, and the inner member is coupled to the frame via the clamp of Fig. 28A.
Fig. 30B is an enlarged view of region 30B from Fig. 30A.
Fig. 31 is a view in perspective of a commissure attached to the clamp of Fig. 27A, according to some embodiments.
Fig. 32A is a view in perspective of the expansion and locking mechanism of Fig. 29B, wherein the outer member is coupled to the frame via the clamp of Fig. 27A having the clamp axial arms oriented in a distal direction.
Fig. 32B is an enlarged view of region 32B from Fig. 32A.
Fig. 33 is a view in perspective of a C-shaped clamp provided with clamp axial arms, according to some embodiments.
Fig. 34A is a view in perspective of the expansion and locking mechanism provided with a tubular outer member, wherein both the outer member and the inner members are coupled to the frame via C-shaped clamps, according to some embodiments.
Fig. 34B is an enlarged view of region 34B from Fig. 34A.
Fig. 34C is an enlarged view of region 34C from Fig. 34A.

### DETAILED DESCRIPTION OF SOME EMBODIMENTS

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present, or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the terms "have" or "includes" means "comprises." As used herein, "and/or" means "and" or "or," as well as "and" and "or".

Directions and other relative references may be used to facilitate discussion of the drawings and principles herein, but are not intended to be limiting. For example, certain terms may be used such as "inner," "outer," "upper," "lower," "inside," "outside,", "top," "bottom," "interior," "exterior," "left," right," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same.

Throughout the figures of the drawings, different superscripts for the same reference numerals are used to denote different embodiments of the same elements. Embodiments of the disclosed devices and systems may include any combination of different embodiments of the same elements. Specifically, any reference to an element without a superscript may refer to any alternative embodiment of the same element denoted with a superscript. In order to avoid undue clutter from having too many reference numbers and lead lines on a particular drawing, some components will be introduced via one or more drawings and not explicitly identified in every subsequent drawing that contains that component.

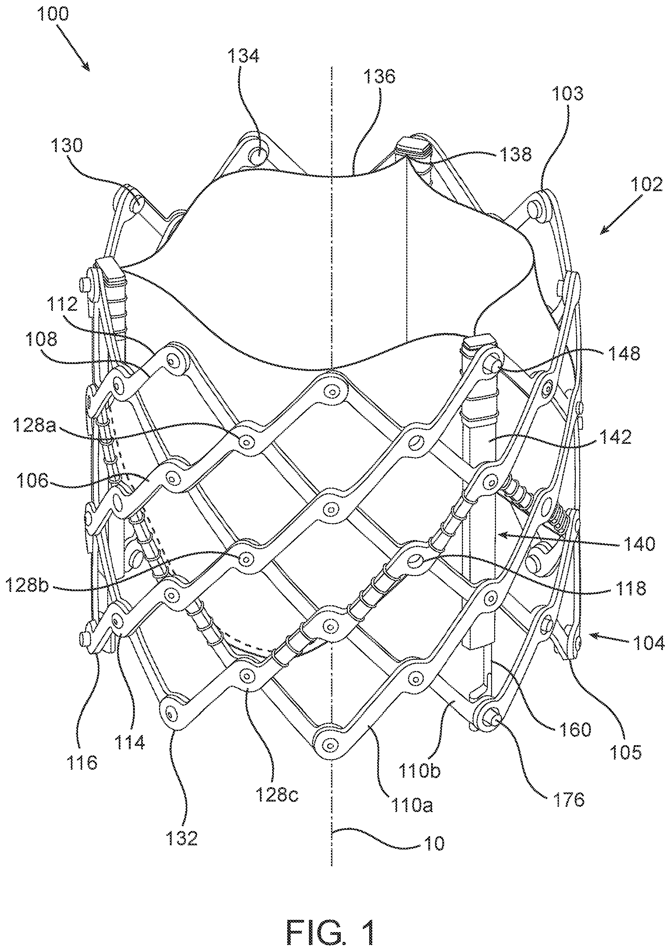

Fig. 1 shows perspective view of an exemplary embodiment of a prosthetic valve 100. The term "prosthetic valve", as used herein, refers to any type of a prosthetic valve deliverable to a patient's target site over a catheter, which is radially expandable and compressible between a radially compressed, or crimped, state, and a radially expanded state. Thus, a prosthetic valve 100 can be crimped or retained by a delivery apparatus (not shown) in a compressed state during delivery, and then expanded to the expanded state once the prosthetic valve 100 reaches the implantation site. The expanded state may include a range of diameters to which the valve may expand, between the compressed state and a maximal diameter reached at a fully expanded state. Thus, a plurality of partially expanded states may relate to any expansion diameter between radially compressed or crimped state, and maximally expanded state. A prosthetic valve 100 of the current disclosure may include any prosthetic valve configured to be mounted within the native aortic valve, the native mitral valve, the native pulmonary valve, and the native tricuspid valve.

The term "plurality", as used herein, means more than one.

Fig. 1 shows a specific exemplary embodiment of a mechanically expandable valve 100, illustrated in an expanded state. Mechanically expandable valves are a category of prosthetic valves that rely on a mechanical actuation mechanism for expansion. The mechanical actuation mechanism usually includes a plurality of expansion and locking assemblies, releasably coupled to respective actuation assemblies of a delivery apparatus, controlled via a handle for actuating the expansion and locking assemblies to expand the prosthetic valve to a desired diameter.

The prosthetic valve 100 can comprise an inflow end portion 104 defining an inflow end 105, and an outflow end portion 102 defining an outflow end 103. The prosthetic valve 100 can define a valve longitudinal axis 10 extending through the inflow end portion 104 and the outflow end portion 102. In some instances, the outflow end 103 is the distal end of the prosthetic valve 100, and the inflow end 105 is the proximal end of the prosthetic valve 100. Alternatively, depending for example on the delivery approach of the valve, the outflow end can be the proximal end of the prosthetic valve, and the inflow end can be the distal end of the prosthetic valve.

The term "proximal", as used herein, generally refers to a position, direction, or portion of any device or a component of a device, which is closer to the user and further away from the implantation site.

The term "distal", as used herein, generally refers to a position, direction, or portion of any device or a component of a device, which is further away from the user and closer to the implantation site.

The term "outflow", as used herein, refers to a region of the prosthetic valve through which the blood flows through and out of the valve 100, for example between the valve longitudinal axis 10 and the outflow end 103.

The term "inflow", as used herein, refers to a region of the prosthetic valve through which the blood flows into the valve 100, for example between inflow end 105 and the valve longitudinal axis 10.

The valve 100 comprises a frame 106 composed of interconnected struts 110, and may be made of various suitable materials, such as stainless steel, cobalt-chrome alloy (e.g. MP35N alloy), or nickel titanium alloy such as Nitinol. According to some embodiments, the struts 110 are arranged in a lattice-type pattern. In the embodiment illustrated in Fig. 1, the struts 110 are positioned diagonally, or offset at an angle relative to, and radially offset from, the valve longitudinal axis 10, when the valve 100 is in an expanded state. It will be clear that the struts 110 can be offset by other angles than those shown in Fig 1, such as being oriented substantially parallel to the valve longitudinal axis 10.

According to some embodiments, the struts 110 are pivotably coupled to each other. In the exemplary embodiment shown in Fig. 1, the end portions of the struts 110 are forming apices 130 at the outflow end 103 and apices 132 at the inflow end 105. The struts 110 can be coupled to each other at additional junctions 128 formed between the outflow apices 130 and the inflow apices 132. Both outflow and inflow apices 130, 132 constitute specific types of junctions 128, but for the sake of simplicity, reference to junction 128 throughout the current specification will refer to intermediate junctions 128 which are not apices 130, 132, but may be rather positioned between the outflow apices 130 and the inflow apices 132, unless stated otherwise.

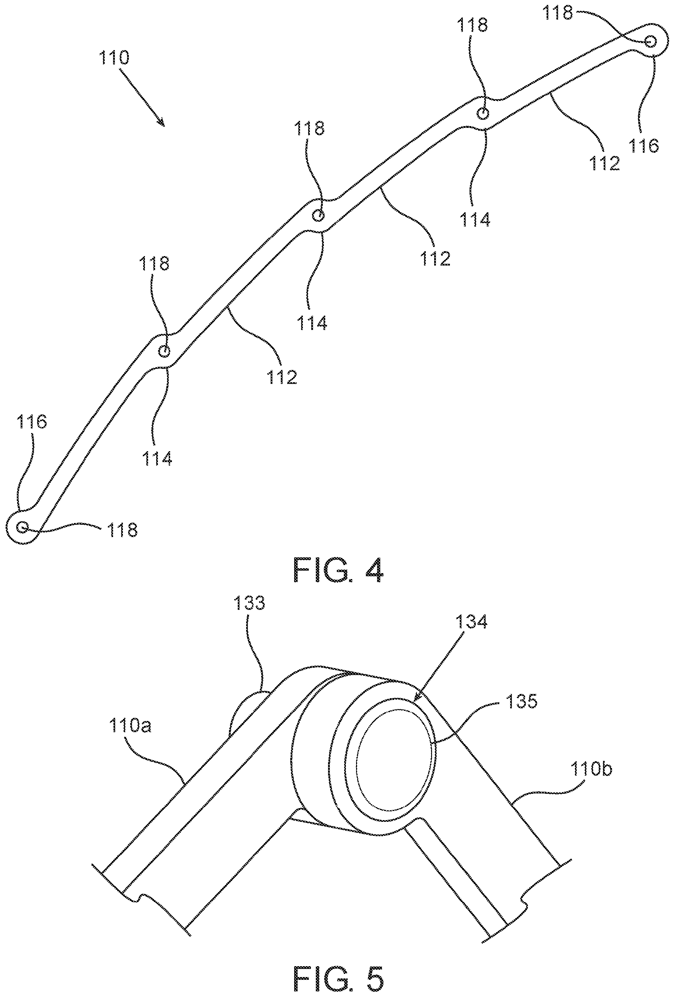

The junctions 128 can be equally spaced apart from each other, and/or from the apices 130, 132 along the length of each strut 110. Frame 106 may comprise openings or apertures 118 (shown in Fig.4) at the regions of apices 130, 132 and junctions 128 of the struts 110. Respective hinges can be included at locations where the apertures 118 of struts 110 overlap each other, via fasteners 134, such as rivets or pins, which extend through the apertures. The hinges can allow the struts 110 to pivot relative to one another as the frame 106 is radially expanded or compressed.

In alternative embodiments, the struts are not coupled to each other via respective hinges, but are otherwise pivotable or bendable relative to each other, so as to permit frame expansion or compression. For example, the frame can be formed from a single piece of material, such as a metal tube, via various processes such as, but not limited to, laser cutting, electroforming, and/or physical vapor deposition, while retaining the ability to collapse/expand radially in the absence of hinges and like.

The frame 106 further comprises a plurality of cells 108, defined between intersecting portions of struts 110. The shape of each cell 108, and the angle between intersecting portions of struts 110 defining the cell borders, vary during expansion or compression of the prosthetic valve 100. Further details regarding the construction of the frame and the prosthetic valve are described in U.S. Publication Nos. 2018/0153689; 2018/0344456; 2019/0060057

A prosthetic valve 100 further comprises one or more leaflets 136, e.g., three leaflets, configured to regulate blood flow through the prosthetic valve 100 from the inflow end 105 to the outflow end 103. While three leaflets 136 arranged to collapse in a tricuspid arrangement, are shown in the exemplary embodiment illustrated in Fig. 1, it will be clear that a prosthetic valve 100 can include any other number of leaflets 136. The leaflets 136 are made of a flexible material, derived from biological materials (e.g., bovine pericardium or pericardium from other sources), bio-compatible synthetic materials, or other suitable materials. The leaflets may be coupled to the frame 106 via commissures 138, either directly or attached to other structural elements connected to the frame 106 or embedded therein, such as commissure posts. Further details regarding prosthetic valves, including the manner in which leaflets may be mounted to their frames, are described in U.S. Patent Nos. 6,730,113, 7,393,360, 7,510,575, 7,993,394 and 8,252,202, and U.S. Patent Application No. 62/614,299,

According to some embodiments, the prosthetic valve may further comprise at least one skirt or sealing member (not shown). An inner skirt can be mounted on the inner surface of the frame (106), configured to function, for example, as a sealing member to prevent or decrease perivalvular leakage. An inner skirt can further function as an anchoring region for the leaflets (136) to the frame (106), and/or function to protect the leaflets (136) against damage which may be caused by contact with the frame (106), for example during valve crimping or during working cycles of the prosthetic valve (100). Additionally, or alternatively, the prosthetic valve (100) can comprise an outer skirt mounted on the outer surface of the frame (106), configure to function, for example, as a sealing member retained between the frame (106) and the surrounding tissue of the native annulus against which the prosthetic valve is mounted, thereby reducing risk of paravalvular leakage past the prosthetic valve (100). Any of the inner skirt and/or outer skirt can be made of various suitable biocompatible materials, such as, but not limited to, various synthetic materials (e.g., PET) or natural tissue (e.g. pericardial tissue).

According to some embodiments, a prosthetic valve 100, which can be a mechanical prosthetic valve, comprises a plurality of expansion and locking assemblies 140, configured to facilitate expansion of the valve 100, and in some instances, to lock the valve 100 at an expanded state, preventing unintentional recompression thereof, as will be further elaborated below. Although Fig. 1 illustrates three expansion and locking assemblies 140, mounted to the frame 106, and optionally equally spaced from each other around an inner surface thereof, it should be clear that a different number of expansion and locking assemblies 140 may be utilized, that the expansion and locking assemblies 140 can be mounted to the frame 106 around its outer surface, and that the circumferential spacing between expansion and locking assemblies 140 can be unequal.

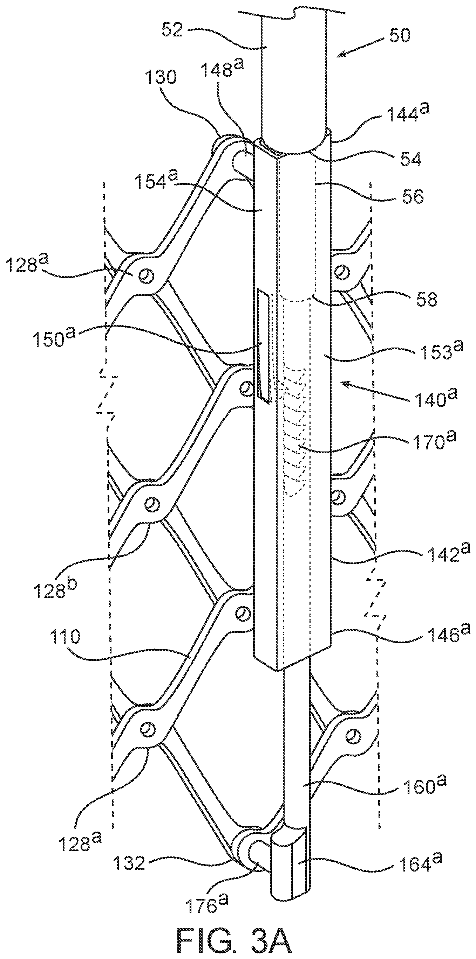

Figs. 3A-3B show an exemplary embodiment of an expansion and locking assembly 140. An expansion and locking assembly 140 may include a hollow outer member 142, secured to a component of the valve (100), such as the frame 106, at a first location, and an inner member 160 secured to a component of the valve (100), such as the frame 106, at a second location, axially spaced from the first location.

Fig. 3A shows a view in perspective of an exemplary embodiment of an inner member 160^{a}, having an inner member proximal end 162^{a} and an inner member distal end portion 164^{a}. The inner member 160^{a} comprises an inner member fastening extension 176^{a} extending from its distal end portion 164^{a}, which may be formed as a pin extending radially outward from the distal end portion 164^{a}, configured to be received within respective openings or apertures 118 of struts 110 intersecting at a junction 128 or an apex 130, 132. The inner member 160^{a} may further comprise a linear rack having a plurality of ratcheting teeth 170^{a} along at least a portion of its length. According to some embodiments, one surface of the inner member 160^{a} comprises a plurality of ratcheting teeth 170^{a}.

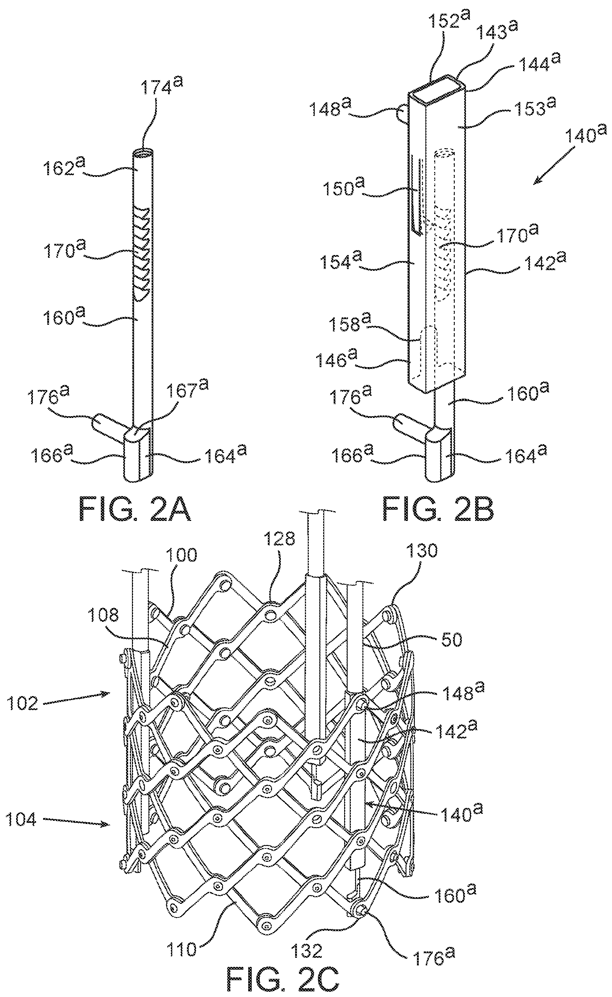

Fig. 2B shows the inner member 160^{a} disposed within a lumen 143^{a} of an exemplary of outer member 142^{a}. The outer member 142^{a} is shown with partial transparency in Fig. 2B for sake of clarity. The outer member 142^{a} comprises an outer member proximal end portion 144^{a} defining a proximal opening of the outer member lumen 143^{a}, and an outer member distal end portion 146^{a} defining a distal opening of the outer member lumen 143^{a}. The outer member 142^{a} can further comprise an outer member fastening extension 148^{a} extending from its proximal end portion 144^{a}, which may be formed as a pin extending radially outward from the external surface of the proximal end portion 144^{a}, configured to be received within respective openings or apertures 118 of struts 110 intersecting at a junction 128 or an apex 130, 132.

The outer member 142^{a} may further comprise a spring biased arm 150^{a}, attached to or extending from one sidewall 154^{a} of the outer member 142^{a}, and having a tooth or pawl at its opposite end, biased inwards toward the inner member 160^{a} when disposed within the outer member lumen 143^{a}.

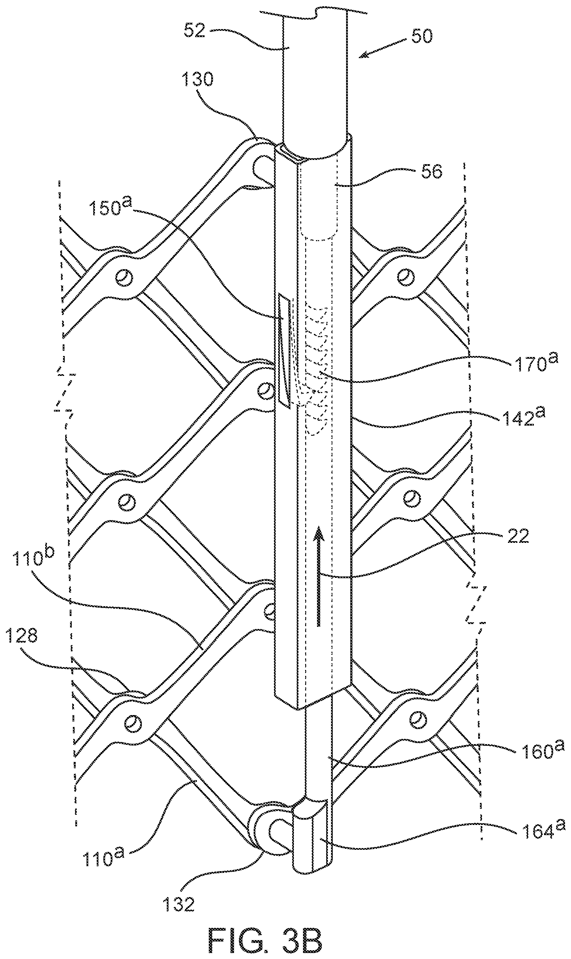

At least one of the inner or outer member 160 or 142, respectively, is axially movable relative to its counterpart. The expansion and locking assembly 140^{a} in the illustrated embodiments, comprises a ratchet mechanism or a ratchet assembly, wherein the pawl of the spring biased arm 150^{a} of the outer member 142^{a} is configured to engage with the teeth 170^{a} of the inner member 160^{a}. The pawl of the spring biased arm 150^{a} can have a shape that is complementary to the shape of the ratcheting teeth 170^{a}, such that the pawl of the spring biased arm 150^{a} allows a sliding movement of the inner member 160^{a} in one direction relative to the outer member 142^{a}, for example in a proximally oriented direction 22 (shown, for example, in Fig. 3B), and resists sliding movement of the inner member 160^{a} in the opposite direction, such as a distally oriented direction, when the pawl of the spring biased arm 150^{a} is in engagement with the ratcheting teeth 170^{a} of the inner member 160^{a}.

The spring biased arm 150^{a} can be formed of a flexible or resilient portion of the outer member 142^{a} that extends over and contacts, via its pawl, an opposing side of the outer surface of the inner member 160^{a}. According to some embodiments, the spring biased arm 150^{a} can be in the form of a leaf spring that can be integrally formed with the outer member 142^{a} or separately formed and subsequently connected to the outer member 142^{a}. The spring biased arm 150^{a} is configured to apply a biasing force against the outer surface of the inner member 160^{a}, so as to ensure that under normal operation, its pawl stays engaged with the ratcheting teeth 170^{a} of the inner member 160^{a}.

The expansion and locking assembly 140 can include, in some embodiments, one or more engagement surfaces configured to prevent over-expansion of the prosthetic valve (100). For example, the outer member distal end portion 146^{a} can include a bore having an outer member engagement surface (not shown). The outer member engagement surface can be configured to engage a corresponding inner member engagement surface 167^{a} to prevent further proximal movement of the inner member 160^{a} relative to the outer member 142^{a}, so as to prevent over expansion of the prosthetic valve (100). As shown in Figs. 2A-2B, the inner member distal end portion 164^{a} can be formed as a wider portion, relative to the remaining portion of the inner member 160^{a} extending proximally therefrom, defining the inner member engagement surface 167^{a} as the proximally-facing surface of the inner member distal end portion 164^{a}.

A mechanically expandable prosthetic valve 100 may be releasably attachable to at least one actuation assembly 50, and preferably a plurality of actuation assemblies 50, matching the number of expansion and locking assemblies 140. The actuation assemblies 50 may be comprised in a delivery apparatus configured to deliver the prosthetic valve (100) to the site of implantation, and facilitate expansion thereof once positioned at the target site. Fig. 2C shows three actuation assemblies 50 coupled to the prosthetic valve 100, however, in will be clear that a greater or fewer number of actuation assemblies 50 can be used in other embodiments.

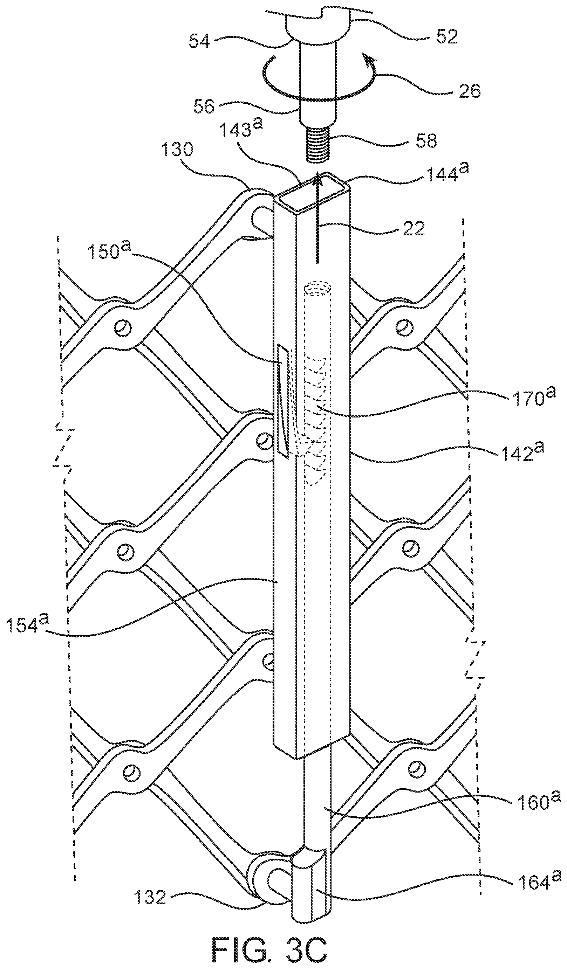

Each actuation assembly 50 can generally include an actuation arm 56 (hidden from view in Fig. 2C, visible in Fig. 3C) releasably coupled at its distal end 58 to respective expansion and locking assembly 140 of the valve 100, and a support sleeve 52 disposed around the respective actuation arm 56. The actuation arm 56 and the support sleeve 52 can be movable longitudinally relative to each other in a telescoping manner to radially expand and contract the frame 106, as further described in U.S. Publication Nos. 2018/0153689, 2018/0153689 and 2018/0325665. The actuation arms 56 can be, for example, wires, cables, rods, or tubes. The support sleeves 52 can be, for example, tubes or sheaths having sufficient rigidity such that they can apply a distally directed force to the frame without bending or buckling.

The inner member proximal end portion 162^{a} further comprises an inner member threaded bore 174^{a}, configured to receive and threadedly engage with a threaded portion of a distal end portion 58 (shown for example in Fig. 3C) of a corresponding actuation arm 56. Fig. 2C shows a view in perspective of a valve 100 in an expanded state, having its expansion and locking assemblies 140^{a} connected to actuation arms 56 (hidden from view within the support sleeves 52) of a delivery apparatus. The leaflets (136), as well as potential fabric or cloth members such as an inner or an outer skirt, are omitted from view in Fig. 2C to expose the expansion and locking assemblies 140^{a} attached to the frame 106. When actuation arms 56 are threaded into the inner members 160^{a}, axial movement of the actuation arms 56 causes axial movement of the inner members 160^{a} in the same direction.

According to some embodiments, the actuation assemblies 50 are configured to releasably couple to the prosthetic valve 100, and to move the prosthetic valve 100 between the radially compressed and the radially expanded configurations. Figs. 3A-3C illustrate a non-binding configuration representing actuation of the expansion and locking assemblies 140^{a} via the actuation assemblies 50 to expand the prosthetic valve 100 from a radially compressed state to a radially expanded state. Fig. 3A shows an expansion and locking assembly 140^{a}, having an outer member 142^{a}, secured to the frame 106 at a first location, and an inner member 160^{a} secured to the frame 106 at a second location. According to some embodiments, the first location can be positioned at an outflow end portion 102, and the second location can be positioned at the inflow end portion 104. In the illustrated embodiment, the outer member 142^{a} is secured to an outflow apex 130 via outer member fastening extension 148^{a}, and the inner member 160^{a} is secured to an inflow apex 132 via inner member fastening extension 176^{a}. A proximal portion of the inner member 160^{a} extends, through the distal opening of the outer member distal end 146^{a}, into the outer member lumen 143^{a}. In some applications, the expansion and locking assemblies 140^{a} or components thereof (e.g., the outer members 142^{a}) may further serve as commissure posts to which commissures 138 may be attached (see Fig. 1).

The expansion and locking assembly 140^{a} is shown in Fig. 3A in a radially compressed state of the valve 100, wherein the outflow and inflow apices 130 and 132, respectively, are relatively distanced apart from each other along the axial direction, and the inner member proximal end 162^{a} is positioned distal to the outer member proximal end 144^{a}.

As further shown in Fig. 3A, the actuation arm distal end portion 58 is threadedly engaged with the inner member threaded bore 174^{a}. According to some embodiments, as shown in Figs. 3A-3C, the actuation arm distal end portion 58 includes external threads, configured to engage with internal threads of the inner member threaded bore 174^{a}. According to alternative embodiments, an inner member may include a proximal extension provided with external threads, configured to be received in and engage with internal threads of a distal bore formed within the actuation arm (embodiments not shown).

The support sleeve 52 surrounds the actuation arm 56 and may be connected to a handle of a delivery apparatus. The support sleeve 52 and the outer member 142^{a} are sized such that the distal lip 54 of the support sleeve 52 can abut or engage the outer member proximal end 144^{a}, such that the outer member 142^{a} is prevented from moving proximally beyond the support sleeve 52.

In order to radially expand the frame 106, and therefore the valve 100, the support sleeve 52 can be held firmly against the outer member 142^{a}. The actuation arm 56 can then be pulled in a proximally oriented direction 22, as shown in Fig. 3B. Because the support sleeve 52 is being held against the outer member 142^{a}, which is connected to an outflow apex 130, the outflow end 103 of the frame 106 is prevented from moving relative to the support sleeve 52. As such, movement of the actuation arm 56 in a proximally oriented direction 22 can cause movement of the inner member 160^{a} in the same direction, thereby causing the frame 106 to foreshorten axially and expand radially.

More specifically, as shown for example in Fig. 3B, the inner member fastening extension 176^{a} extends through apertures 118 in two struts 110 interconnected at an inflow apex 132, while the outer member fastening extension 148^{a} extends through aperture 118 in two struts 110 interconnected at an outflow apex 130. As such, when the inner member 160^{a} is moved axially, for example in a proximally oriented direction 22, within the outer member 142^{a}, the inner member fastening extension 176^{a} moves along with the inner member 160^{a}, thereby causing the portion to which the inner member fastening extension 176^{a} is attached to move axially as well, which in turn causes the frame 106 to foreshorten axially and expand radially.

The struts 110 to which the inner member fastening extension 176^{a} is connected are free to pivot relative to the fastening extension 176^{a} and to one another as the frame 106 is expanded or compressed. In this manner, the inner member fastening extension 176^{a} serves as a fastener that forms a pivotable connection between those struts 110. Similarly, struts 110 to which the outer member fastening extension 148^{a} is connected are also free to pivot relative to the fastening extension 148^{a} and to one another as the frame 106 is expanded or compressed. In this manner, the outer member fastening extension 148^{a} also serves as a fastener that forms a pivotable connection between those struts 110.

When the pawl of the spring biased arm 150^{a} is engaged with the ratcheting teeth 170^{a}, the inner member 160^{a} can move in one axial direction, such as the proximally oriented direction 22, but cannot move in the opposite axial direction. This ensures that while the pawl of the spring biased arm 150^{a} is engaged with the he ratcheting teeth 170^{a}, the frame 106 can radially expand but cannot be radially compressed. Thus, after the prosthetic valve (100) is implanted in the patient, the frame 106 can be expanded to a desired diameter by pulling the actuation arm 56. In this manner, the actuation mechanism also serves as a locking mechanism of the prosthetic valve (100).

Once the desired diameter of the prosthetic valve (100) is reached, the actuation arm 56 may be rotated in direction 26 to unscrew the actuation arm 56 from the inner member 160^{a}, as shown in Fig. 3C. This rotation serves to disengage the distal threaded portion 58 of the actuation arm 56 from the inner member threaded bore 174^{a}, enabling the actuation assemblies 50 to be pulled away, and retracted, together with the delivery apparatus, from the patient's body, leaving the prosthetic valve (100) implanted in the patient. The patient's native anatomy, such as the native aortic annulus in the case of transcatheter aortic valve implantation, may exert radial forces against the prosthetic valve (100) that would strive to compress it. However, the engagement between the pawl of the spring biased arm 150^{a} and the ratcheting teeth 170^{a} of the inner member 160^{a} prevents such forces from compressing the frame 106, thereby ensuring that the frame 106 remains locked in the desired radially expanded state.

Thus, the prosthetic valve 100 is radially expandable from the radially compressed state shown in Fig. 3A to the radially expanded state shown in Fig. 3B upon actuating the expansion and locking assemblies 140^{a}, wherein such actuation includes approximating the second locations to the first locations of the valve. The prosthetic valve 100 is further releasable from the delivery apparatus by decoupling each of the actuation assemblies 50 from each of the corresponding expansion and locking assemblies 140^{a} that were attached thereto.

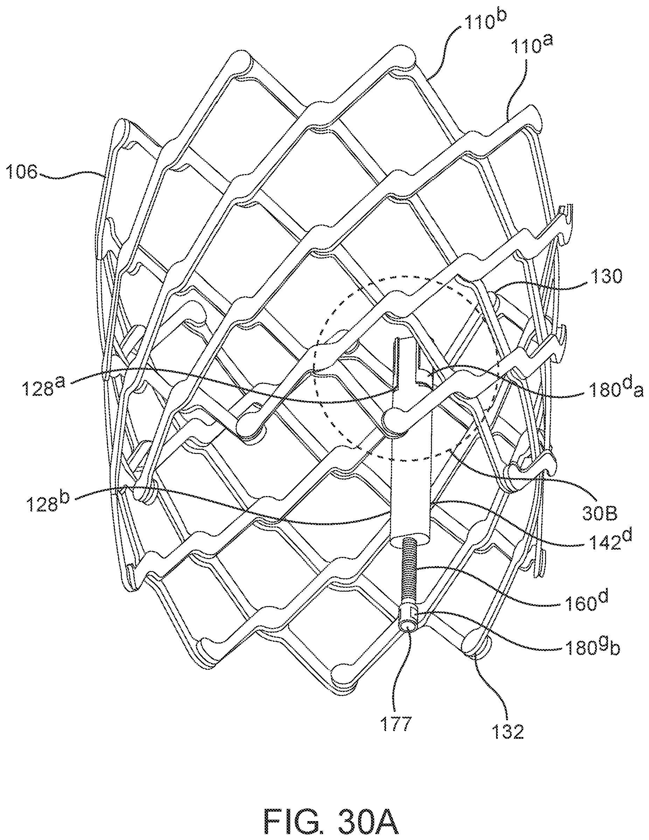

While the inner member 160^{a} and the outer member 142^{a} are shown in the illustrated embodiment connected to an inflow apex 132 and an outflow apex 130, respectively, it should be understood that they can be connected to other junctions 128 of the frame 106. For example, the inner member fastening extension 176^{a} can extend through aperture 118 formed in interconnected struts 110 at a junction 128c at the inflow end portion 104, proximal to the inflow apices 132 (similar to a configuration shown in Fig. 30A, for example). Similarly, the outer member fastening extension 148^{a} can extend through apertures 118 formed in interconnected struts at a junction 128a at the outflow end portion 102, distal to the outflow apices 130 (also similar to a configuration shown in Fig. 30A, for example).

While the frame 106 is shown above to expand radially outward by axially moving the inner member 160 in a proximally oriented direction 22, relative to the outer member 142, it will be understood that similar frame expansion may be achieved by axially pushing an outer member 142 in a distally oriented direction, relative to an inner member 160. Moreover, while the illustrated embodiments show the outer member 142 affixed to an outflow end portion 102 of the frame 106, and an inner member 160 affixed to an inflow end portion 104 of the frame 106, in alternative embodiments, the outer member 142 may be affixed to the inflow end portion 104 of the frame 106, while the inner member 160 may be affixed to the outflow end portion 102 of the frame 106.

While a threaded engagement is illustrated and described in the above embodiments, serving as an optional reversible-attachment mechanism between the actuation assemblies 50 and the inner members 160, it is to be understood that in alternative implementations, other reversible attachment mechanisms may be utilized, configured to enable the inner member 160 to be pulled or pushed by the actuation assemblies 50, while enabling disconnection there-between in any suitable manner, so as to allow retraction of the delivery apparatus from the patient's body at the end of the implantation procedure.

The terms coupled, engaged, connected and attached, as used herein, are interchangeable. Similarly, the term decoupled, disengaged, disconnected and detached, as used herein, are interchangeable.

While a specific actuation mechanism is described above, utilizing a ratcheting mechanism between the inner and the outer members of the expansion and locking assemblies 140, other mechanisms may be employed to promote relative movement between inner and outer members of actuation assemblies, for example via threaded or other engagement mechanisms. Further details regarding the structure and operation of mechanically expandable valves and delivery system thereof are described in US Patent No. 9,827,093, U.S. Patent Application Publication Nos. 2019/0060057, 2018/0153689 and 2018/0344456, and US Patent Application Nos. 62/870,372 and 62/776,348,

FIG. 4 shows a representative embodiment of a strut 110. A plurality of such struts 110 can be arranged in a lattice pattern to form a frame 106. Each strut 110 can have an offset, or zig-zag, pattern defined by a plurality of offset linear segments 112. In the illustrated embodiment, each segment 112 of the strut 110 is curved such that the overall shape of the strut 110 is curved. As shown, the linear segments 112 can be arranged end-to-end relative to each other with adjacent ends interconnected to each other by intermediate segments 114. The strut 110 can have enlarged end portions 116 that form the apices at the inflow and outflow 132, 130 of the frame 106. Each of the intermediate segments 114 and strut end portions 116 can have a respective aperture 118, such as at its geometric center, for receiving a fastener 134. Each linear segment 112 may be slightly laterally offset from an adjacent linear segment 112 in a direction perpendicular to the overall length of the strut 110 to provide the zig-zag pattern to the strut. In alternative embodiments, the segments 112 can be arranged without any offset relative to each other.

With reference to Fig. 5, the plurality of struts 110 can include a plurality of radially disposed outer struts 110a and a plurality of radially disposed inner struts 110b. The outer and inner struts 110a, 110b can be pivotably coupled to one another at one or more pivot joints along the length of each strut. Respective hinges or junctions 128 are thus formed at the locations where struts 110 overlap each other (including at apices 130, 132) via fasteners 134 that extend through the apertures 118. The junctions 128 can allow the struts 110 to pivot relative to one another as the frame 106 is radially expanded or compressed, such as during assembly, preparation, or implantation of the prosthetic valve (100).

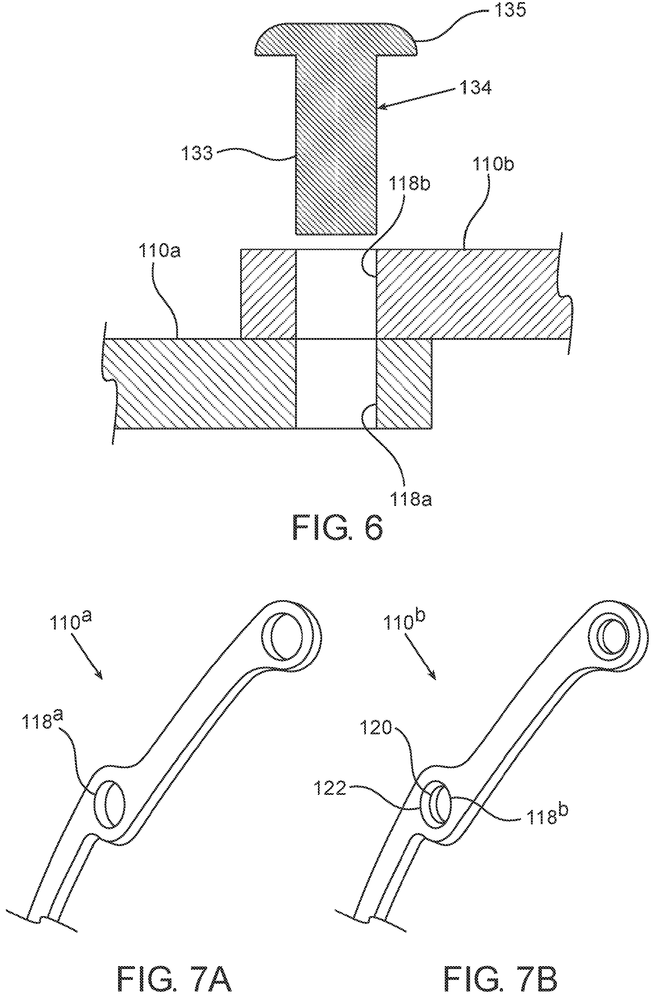

With reference to Fig. 6, the apertures 118 can be used to connect the outer and inner struts 110a, 110b to one another using fasteners 134, such as rivets or pins. Each fastener 134 can be formed with a fastener main body 133, which may be shaped as an elongated longitudinal shaft, and an enlarged fastener head 135. The inner diameter of the apertures 118a, 118b can be closely matched to that of the fastener main body 133, configured to receive the fastener main body 133 therethrough. The diameter of the fastener head 135, as shown in Fig. 6, can be larger than that of the apertures 118a, 118b, enabling the fastener head 135 to abut the surface of the strut 110 surrounding the aperture 118.

In some implementations, the length of the fastener main body 133 can be shorter than or equal to the length of both apertures 118a and 118b when aligned with each other. In some implementations, as shown in Fig. 5, the length of the fastener main body 133 can be longer than the length of both apertures 118a and 118b when aligned with each other, enabling it to protrude beyond the struts 110, such that an end cap or a nut (not shown) can be attached to the protruding end of the fastener main body 133 to secure the fastener 134 in position. In some implementations, a protruding end portion of the fastener main body 133 can be flanged, for example by the use of a die or any other suitable tool, to secure the fastener 134 in position.

Fig. 6 shows an optional arrangement, in which the fastener heads 135 are positioned radially inward, for example, abutting the inner struts 110b, while the fastener main bodies 133 extend radially outward, toward and through the outer struts 110a. In alternative arrangements, the fastener heads 135 may be positioned radially outward, for example, abutting the outer struts 110a, while the fastener main bodies 133 extend radially inward, toward and through the inner struts 110b.

While both inner 110b and outer 110a struts are shown in the illustrated embodiments, such as for example in Fig. 6, as being tightly positioned over each other, in reality, spacers such as washers or bushings (not shown) can be disposed at a junction 128 between the inner and outer struts 110b, 110a. Such spacers can assist the struts 110 in moving relative to one another. In other implementations, the struts 110 can be fixedly connected to one another, such as by welding or adhesion, or by laser-cutting the individual struts of the frame from a metal tube. Further details of the struts can be found in U.S. Patent Application Publication No. 2018/0344456,

In some implementations, the inner struts 110b and the outer struts 110a may be provided as structurally identical struts, having the same dimensions and overall shape and design, as shown in Fig. 6. However, in alternative implementations, the inner struts 110b and the outer struts 110a can each have unique features.

With reference to Figs. 7A-7B, the outer struts 110a may differ from the inner struts 110b by the shape of the apertures 118 configured to receive different portions of the fastener 134. Fig. 7A shows one type of a strut 110^{a}, which includes apertures 118^{a} having a uniform diameter along their lengths. Fig. 7B shows another type of a strut 110^{b}, wherein the apertures 118^{b} include a main bore 120, having a diameter similar to that of the apertures 118^{a}, configured to accommodate the fastener main body 133 therein, and a wider counter-bore 122 configured to accommodate the fastener head 135 therein.

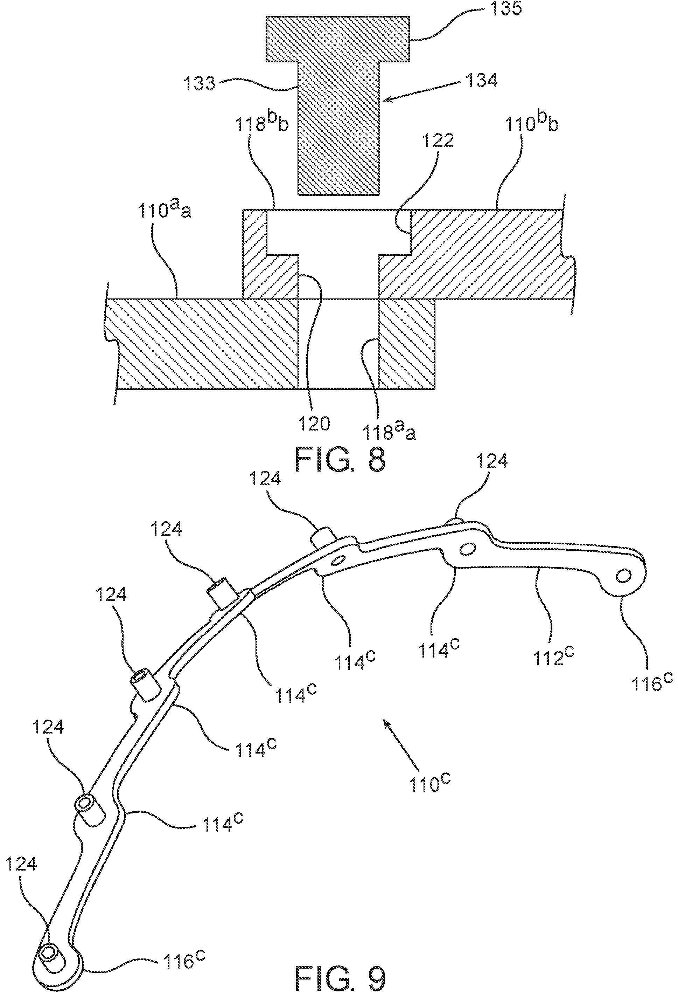

With reference to Fig. 8, the inner struts 110^{b}b can be provided with apertures 118^{b}b that include counter-bores 122, into which the fasteners head 135 may be inserted, while the fastener main bodies 133 may extend through the main bores 120 of the inner apertures 118^{b}b and through the outer apertures 118^{a}a. Advantageously, the counter-bore 122 may accommodate the fastener head 135 such that the fastener head 135 is either flush with the surface of the inner surface of the strut 110^{b}b, or may be even inserted further inside the 118^{b}b, such that in either case, the fastener head 135 will not protrude from the apertures 118 of junctions 128 and/or apices 130, 132.

Fig. 8 shows an optional arrangement, in which the inner struts 110b are of the type that includes apertures 118^{b} with counter-bores 122, while the outer struts 110a are provided with uniformly shaped apertures 118^{a}. In alternative arrangements, the outer struts 110a are of the type that includes apertures 118^{b} with counter-bores 122, while the inner struts 110b are provided with uniformly shaped apertures 118^{a}. In yet other alternative arrangements, both the inner struts 110b and the outer struts 110a are provided with apertures 118^{b} having counter-bores 122. In such cases, the dimensions of the counter-bores 122 may differ between the inner 110b and outer 110a struts, wherein the counter-bores 122 on one side may be dimensioned to receive the fastener heads 135, and the counter-bores 122 on the opposite side are dimensioned to accommodate flanged portions at the ends of the fastener main bodies 133 (not shown).

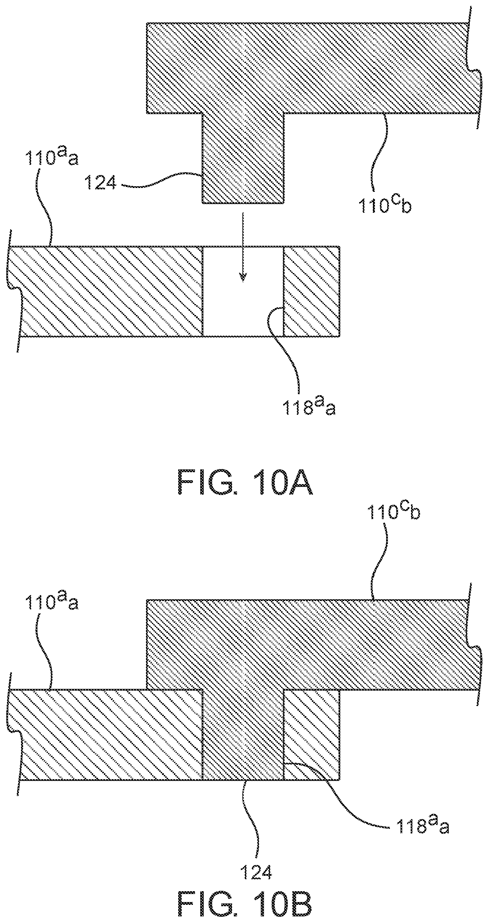

Fig. 9 shows another type of a strut 110^{c} provided with a plurality of integral fasteners 124 extending from the central regions of the intermediate segments 114^{c} and/or the strut end portions 116^{c}, instead of apertures (118). Figs. 10A and 10B show a strut 110^{c} with integral fasteners 124 and a strut 110^{a} provided with apertures 118^{a}, before and after being engaged together, respectively. The diameter of the apertures 118^{a} can be closely matched to that of the integral fasteners 124, configured to accommodate the integral fasteners 124 therein.

In some implementations, the length of the integral fastener 124 can be shorter than or equal to the length of the aperture 118^{a} (see Fig. 10B). In other implementations, the length of the integral fastener 124 can be longer than the length of the apertures 118^{a}, enabling it to protrude beyond the strut 110^{a}, such that an end cap or a nut (not shown) can be attached to the protruding end of the integral fastener 124 to secure the integral fastener 124 in position. In some implementations, a protruding end portion of the integral fastener 124 can be flanged, for example by the use of a die or any other suitable tool, to secure the integral fastener 124 in position.

Figs. 10A-10B shows an optional arrangement, in which the inner struts 110b are of the type that includes integral fasteners 124, while the outer struts 110a are provided with uniformly shaped apertures 118^{a}. In alternative arrangements, the outer struts 110a are of the type that includes integral fasteners 124, while the inner struts 110b are provided with uniformly shaped apertures 118^{a}.

Advantageously, utilization of struts 110^{c} that include integral fasteners 124 can significantly simplify, and speed up, the process of assembling a frame 106 of a prosthetic valve 100, by eliminating the need to spend time and effort to connect inner and outer struts via a large number of individual separate fasteners.

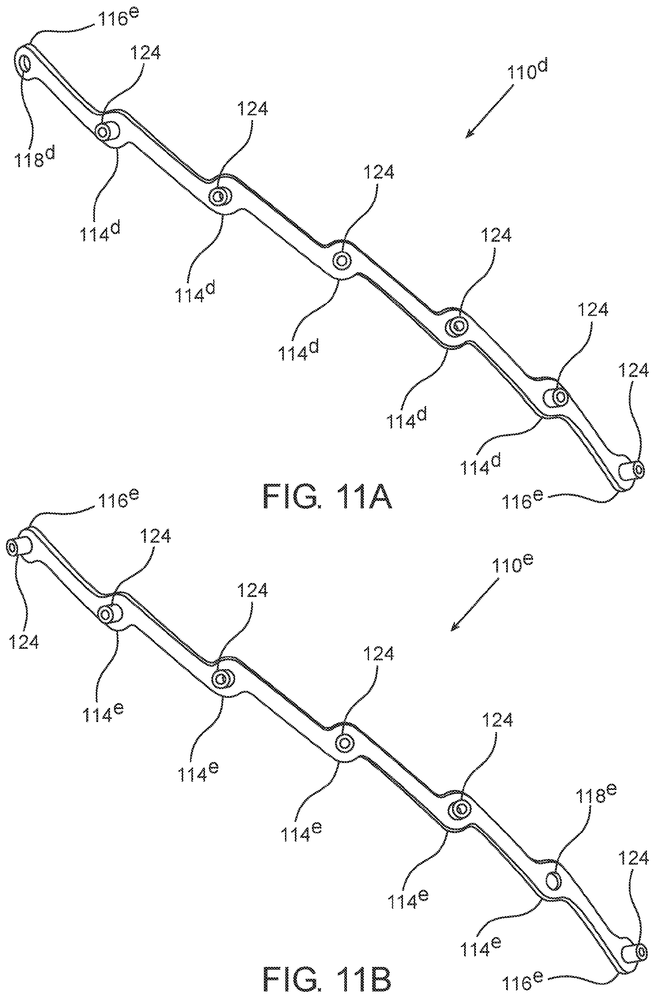

Figs. 11A-11B shows additional types of struts 110, which are similar to strut 110^{c} having at least one integral fastener 124, and preferably a plurality of integral fasteners 124, as well as at least one aperture 118. Fig. 11A shows a type of a strut 110^{d} provided with a plurality of integral fasteners 124, similar to fastener 134, but differing in that at least one of the strut end portions 116^{d} includes an aperture 118^{d} instead of an integral fastener 124. While the strut 110^{d} is illustrated in Fig. 11A as having a single aperture 118^{d} at one strut end portion 116^{d}, wherein the opposite end portion 116^{d} is provided with an integral fastener 124, in other implementations, the strut 110^{d} can include apertures 118^{d} at both of its end portions 116. The apertures 118^{d} can be uniformly shaped, similar to apertures 118^{a}, or may be provided with counter-bores 122, similar to apertures 118^{b}.

Fig. 11B shows a type of a strut 110^{e} provided with a plurality of integral fasteners 124, similar to fastener 134, but differing in that at least one of the intermediate segments 114^{e} includes an aperture 118^{e} instead of an integral fastener 124. While the strut 110^{e} is illustrated in Fig. 11B as having a single aperture 118^{e} at one intermediate segment 114^{e}, in other implementations, the strut 110^{e} can include apertures 118^{e} within several intermediate segments 114^{e}. The apertures 118^{e} can be uniformly shaped, similar to apertures 118^{a}, or may be provided with counter-bores 122, similar to apertures 118^{b}.

The implementations shown for strut 110^{d} and strut 110^{e} can be also combined, such that a single strut will include at least one integral fastener 124, at least one aperture 118 extending through either a single strut end portion 116 or both opposite end portions 116, and at least one aperture 118 extending through at least one intermediate segment 114.

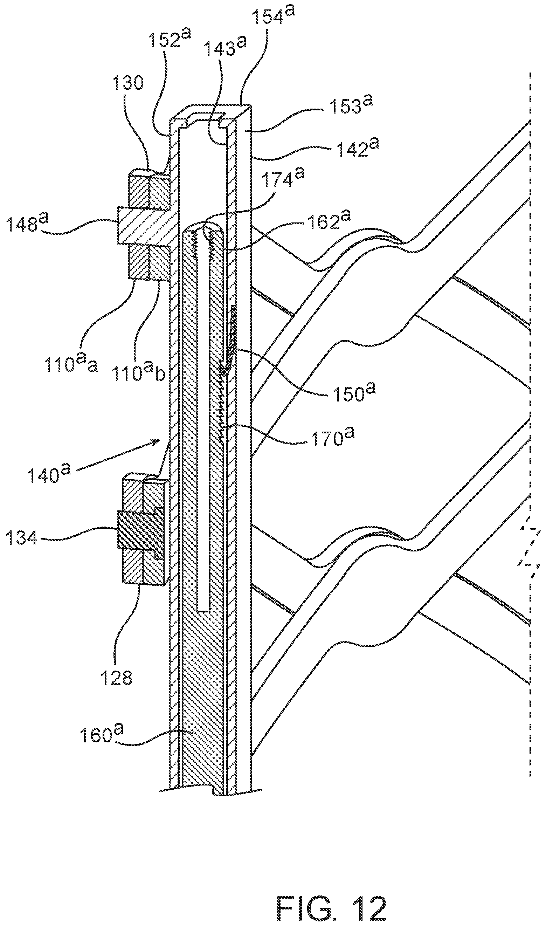

With reference to Fig. 12, an expansion and locking assembly 140^{a} can be coupled to a frame 106 by insertion of its fastening extensions, such as the outer member fastening extensions 148^{a} shown in Fig. 12, and such as the inner member fastening extension 176^{a} (not visible in Fig. 12), through respective apertures 118 of the inner struts 110b and the outer struts 110a. The inner and outer struts 110b, 110a at the remainder of the junctions 128 or apices 130, 132, which are not positioned at locations of expansion and locking assembly (140^{a}) attachment to the frame (106), may be joined together via fasteners 134.

When struts 110^{c} having a plurality of integral fasteners 124 are to be utilized, the integral fasteners 124 at the junctions 128 to which an expansion and locking assembly (140^{a}) needs to be attached, may interfere with the outer member fastening extensions 148^{a} and/or the inner member fastening extension 176^{a}. Thus, struts 110^{d} of 110^{e} may be required for assembling a frame 106, to which expansion and locking assemblies 140^{a} provided with outer member fastening extensions 148^{a} and/or inner member fastening extension 176^{a} need to be attached.

For example, an outflow apex 130 to which an expansion and locking assembly 140^{a} needs to be attached, in an arrangement similar to that shown in Fig. 12, *mutatis mutandis,* may comprise an outer strut 110^{a}a provided with apertures 118^{a}a, and an inner strut 110^{d}b provided with a plurality of integral fasteners 124 that may be inserted in a relatively fast and simple manner into the apertures 118^{a}a, and with an aperture 118^{d}b at the outflow junction 130, allowing the outer member fastening extensions 148^{a} to extend through the apertures 118^{d}b and 118^{a}a. If the opposite end portion 116^{d} of the same inner strut 110^{d}b is positioned at an inflow apex 132 to which an inner member 160^{a} of a different expansion and locking assembly 140^{a} is to be attached, the same inner strut 110^{d}b may include an aperture 118^{d}b extending through its opposite end portion 116^{d} as well.

In other implementations, the expansion and locking assembly 140^{a} may not necessarily be attached to the apices 130, 132 of the frame 106, but rather to other junctions 128, such as junctions 128a which are distal to the outflow apices 130, and junctions 128c which are proximal to the inflow apices 132 (similar to the arrangement shown in Fig. 30A, *mutatis mutandis*)*.* In such cases, struts 110° may be utilized in a similar manner to that describe above for struts 110^{d}, wherein the apertures 118^{e}b of the inner struts 110^{e}b are positioned at the junctions of expansion and locking assembly (140^{a}) attachment to the frame (106), allowing the outer member fastening extensions 148^{a}, and/or the inner member fastening extension 176^{a}, to extend through the apertures 118^{e}b and 118^{a}a.

While utilization of struts 110 having integral fasteners 124 may significantly simplify attachment between inner and outer struts at most of the junctions 128 and apices 130, 132, the requirement to adjust some of the struts to include apertures 118 instead of integral fasteners 124 at some of the extending through some of their end portions 116 and/or intermediate segments 114, may pose a manufacturing challenge, as in such cases, the same frame 106 may be comprised of various types of different struts 110. For example, the frame 106 can include outer struts 110^{a}a provided with apertures 118^{a}a, and more than one type of inner struts 110b, such as inner struts 110^{c}b provided only with integral fasteners 124, that can be joined to outer struts 110^{a}a along junctions 128 and apices 130, 132 that are not coupled to expansion and locking assemblies (140^{a}), as well as additional types of inner struts 110^{d}b and/or 110^{e}b, to accommodate respective outer member fastening extensions 148^{a} and/or the inner member fastening extension 176^{a}. The requirement for different types of struts 110 can significantly increase manufacturing costs. Thus, it may be beneficial from both a manufacturing and assembling labor standpoint, to provide a more efficient and cost-effective solution for assembling a valve with expansion and locking assemblies attachable thereto.

According to some embodiments, a prosthetic valve 100 comprises at least one clamp 180, coupled to the frame 106, wherein at least one expansion and locking assembly 140 is configured to snap-fit or clip into the at least one clamp 180.

The at least one clamp 180 can include a first clamp 180a, coupled to the frame at a first location, and a second clamp 180b, coupled to the frame at a second location spaced apart from the first location. The expansion and locking assembly (140) may be attached to the frame (106) via the at least one clamp 180, for example such that the outer member (142) is attached to the first location via the first clamp 180a, and the inner member (160) is attached to the second location via the second clamp 180b. In some configurations, the first location may be an outflow apex 130 or another non-apical junction 128a at the outflow end portion 102, and the second location may be an inflow apex 132 or another non-apical junction 128c at the inflow end portion 104. In other configurations, the first location may be an inflow apex 132 or another non-apical junction 128c at the inflow end portion 104, and the second location may be an outflow apex 130 or another non-apical junction 128a at the outflow end portion 102.

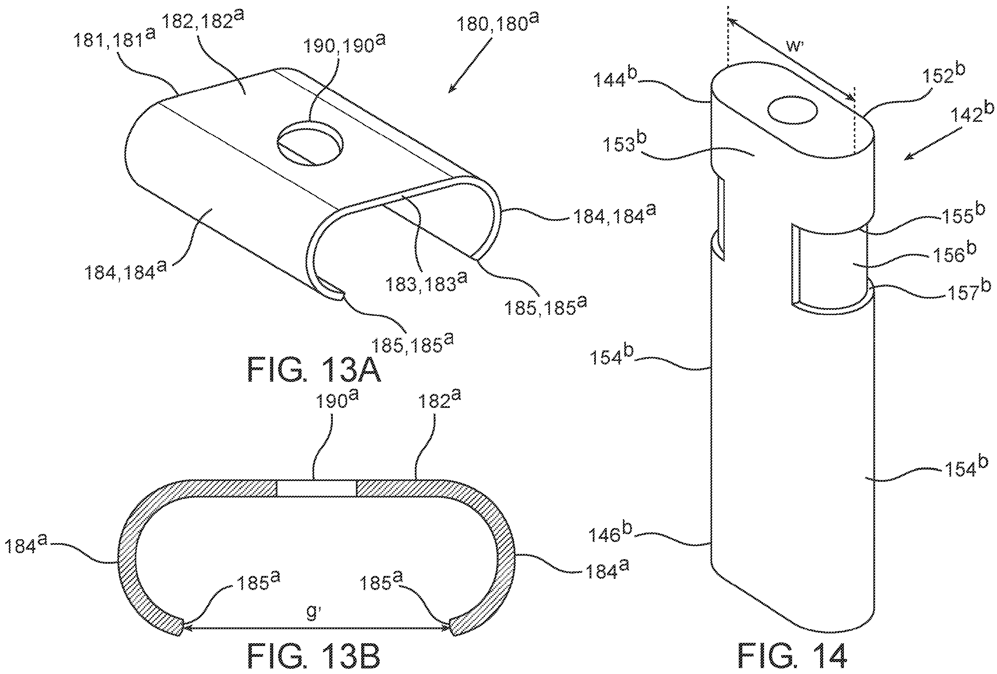

With reference to Figs. 13A-13B, the clamp 180 comprises a clamp mid-portion 182, and a couple of inwardly biased opposing side arms 184 extending in a continuous manner from both sides of the mid-portion 182 and terminating at free ends 185. As illustrated, the side arms 184 may be arched inward, defining a gap g' between their free ends 185, through which an element, such as a component of the expansion and locking assembly 140, may pass toward the mid-portion 182. In some implementation, the clamp 180 if formed as a single monolithic component, for example formed from a single plate, having both sides thereof bent to form the side arms 184. In alternative implementations, the clamp 180 may be comprised of more than one component, for example by having two separately formed side arms that may be affixed to two opposing edges of a mid-portion.

According to some embodiments, the clamp mid-portion 182 comprises an opening 190, configured to receive a fastener that may extend therethrough, such as a fastener 134, an outer member fastening extension 148, or an inner member fastening extension 176. Figs. 13A-13B illustrate an exemplary embodiment of a clamp 180^{a} comprising an opening 190^{a} extending through the thickness of the clamp mid-portion 182^{a}. The side arms 184^{a} are continuously arching from the clamp mid-portion 182^{a} toward each other at their opposite free ends 185^{a}, defining a gap g' between the free ends 185^{a}. In some implementations, such as that of clamp 180^{a} shown in Figs. 13A-13B, the clamp mid-portion 182^{a} may be substantially flat.

According to some embodiments, the expansion and locking assembly 140 comprises at least one coupling recess, configured to accommodate at least a portion of the side arms 184 of a clamp 180, and in some implementations, at least a portion of the mid-portion 182 of the clamp 180.

In some implementations, an outer member 142 can include an outer member coupling recess 156. The outer member 142 comprises an outer wall 152 configured to face the frame (106), an opposite inner wall 153 configured to face the valve longitudinal axis 10, and two sidewalls 154. The outer member coupling recess 156 spans across the sidewalls 154 of the outer member 142, and optionally along at least a portion of the inner wall 153. Optionally, but not necessarily, the outer member coupling recess 156 may also span across the outer wall 152.

Fig. 14 shows an exemplary embodiment of an outer member 142^{b}, comprising an outer member coupling recess 156^{b} configured to accommodate at least a portion of a clamp 180 therein. The outer member coupling recess 156^{b} shown in Fig. 14 is shaped so as to closely match the shape of the clamp 180^{a} shown in Figs. 13A-13B. The outer wall 152^{b} may be provided as a generally flat wall, while the sidewalls 154^{b} may be rounded. The inner wall 153^{b} may be flat and parallel to the outer wall 152^{b}. In some implementations, as shown in Fig. 14, the outer member coupling recess 156^{b} spans across the outer wall 152^{b}, configured to accommodate the clamp mid-portion 182^{a}, and across the sidewalls 154^{b}, configured to accommodate the side arms 184^{a}. Optionally, but not necessarily, the outer member coupling recess 156^{b} may also span across a portion of the inner wall 153^{b}, configured to accommodate end portions of the side arms 184^{a}.

The side arms 184 are resiliently expandable away from each other, such that the outer member coupling recess 156 is passable through the gap g' formed between their free ends 185. In use, the outer member 142 may be pushed into the clamp 180, having the outer member coupling recess 156 aligned with the clamp 180. The gap g' is smaller than the maximal lateral width w' of the outer member 142 (width w' may be measured at the region of the outer member coupling recess 156). The sidewalls 154 of the outer member 142, at the region of the recess 156, may apply a force sufficient to expand the side arms 184 away from each other, so as to allow the outer member 142 to pass therethrough radially outward, toward the clamp mid-portion 182. Once the outer member coupling recess 156 is completely accommodated within the clamp 180, and in the absence of further expanding force applied to the side arms 184, the side arms resiliently snap back toward each other to compress against the outer member coupling recess 156, in order to lock in place the outer member 142 with respect to the clamp 180.

The outer member coupling recess 156 is defined between a recess proximal edge 155 (such as recess proximal edge 155^{b} shown in Fig. 14) and a recess distal edge 157 (such as recess distal edge 157^{b} shown in Fig. 14). In some implementations, the height of the outer member coupling recess 156, defined as the distance between the recess proximal edge 155 and a recess distal edge 157, is substantially equal to the longitudinal length of the clamp 180, such that when the outer member 142 is engaged with the clamp 180, the clamp proximal edge 181 and clamp distal edge 183 (shown, for example, in Fig. 13A) abut the recess proximal edge 155 and the recess distal edge 157, respectively, thereby preventing axial movement in any direction of the outer member 142 with respect to the clamp 180.

The term "substantially equal", as used herein, means within a range of no more than ±10 percent of the referred measure. For example, the height of the outer member coupling recess 156 being substantially equal to the longitudinal length of the clamp 180, means that this height is not greater than 110% of the longitudinal length of the clamp 180.

In some implementations, the depth of the outer member coupling recess 156 is substantially equal to the thickness of the clamp 180, such that when the clamp 180 is engaged with the outer member 142, the clamp's outer surface is flush with the outer surface of the outer member (142) around the recess 156.

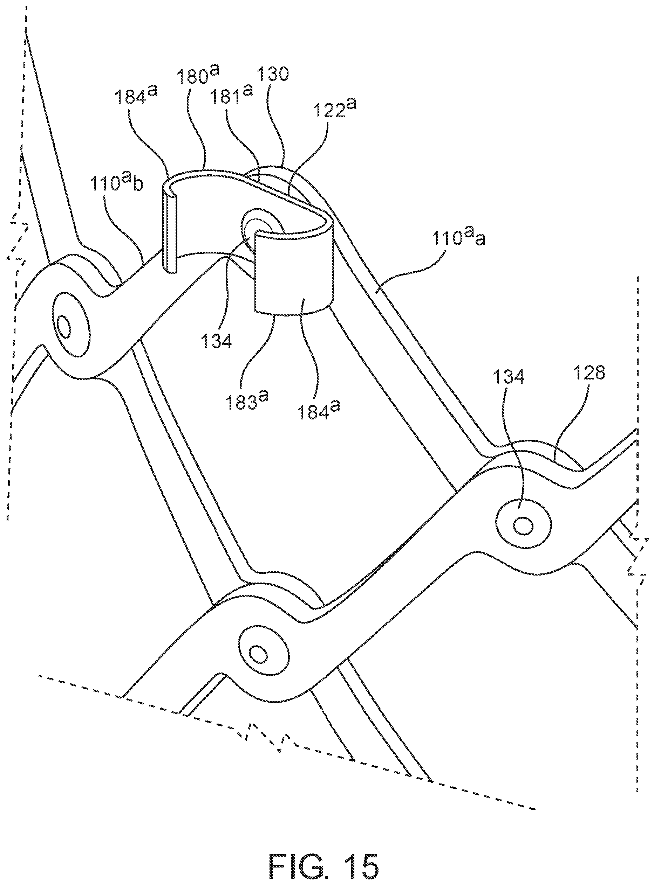

In some embodiments, an outer member 142^{b} comprising an outer member coupling recess 156^{b}, may be provided without an integral outer member fastening extension (148). With reference to Fig. 15, a clamp 180^{a} can be attached to the struts 110 of a frame 106 via a fastener 134, which will enable coupling of the outer member 142^{b} to the frame 106 via the clamp 180^{a}, by snapping it into the clamp 180^{a} as described hereinabove. While the clamp 180^{a} is shown in Fig. 15 coupled to an outflow apex 130, it will be clear that it may be coupled in the same manner to an inflow apex 132, or to any other non-apical junction 128.

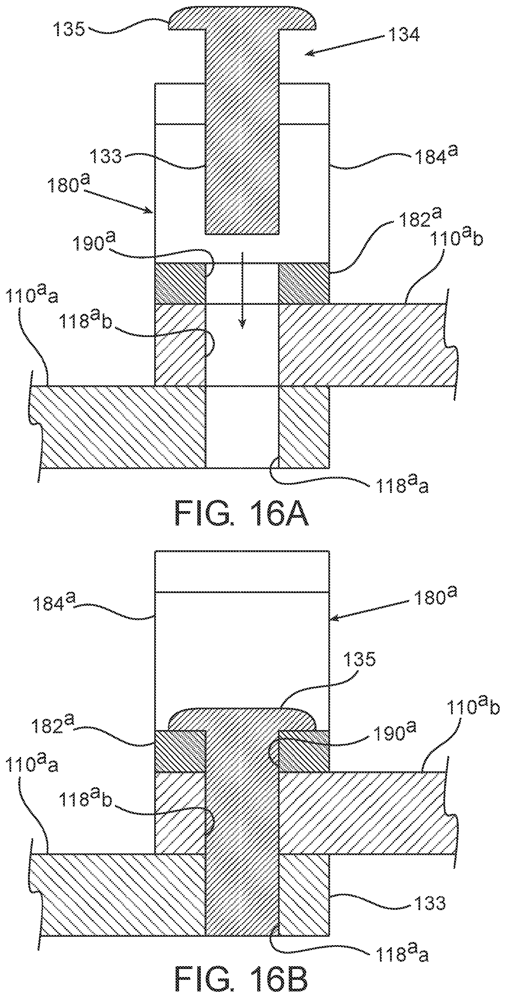

Figs. 16A and 16B show the clamp 180^{a}, along with an inner strut 110^{a}b and an outer strut 110^{a}a, as shown in Fig. 15, before and after being engaged together, respectively. As shown in the illustrated configuration, both the inner strut 110^{a}b and the outer strut 110^{a}a are provided with an inner aperture 118^{a}b and an outer aperture 118^{a}a, respectively, at the junction to which the clamp 180^{a} is coupled. The opening 190^{a} of the clamp is aligned with the inner aperture 118^{a}b and the outer aperture 118^{a}a, and may be preferably sized to have a diameter substantially equal to that of the inner aperture 118^{a}b and the outer aperture 118^{a}a, matching the diameter of a fastener main body 133. As shown, a fastener 134 may be inserted through the opening 190^{a} and the apertures 118^{a}b, 118^{a}a, to secure the clamp 180^{a} and both inner and outer struts 110^{a}b, 110^{a}a, together.

In some implementations, the length of the fastener main body 133 can be shorter than or equal to the combined thickness of the outer strut 110^{a}a, the inner strut 110^{a}b, and the clamp mid-portion 182^{a}, when aligned with each other. In other implementations, the length of the fastener main body 133 can be longer than the combined thickness of the outer strut 110^{a}a, the inner strut 110^{a}b, and the clamp mid-portion 182^{a}, when aligned with each other, enabling it to protrude beyond the struts 110, such that an end cap or a nut (not shown) can be attached to the protruding end of the fastener main body 133 to secure the fastener 134 in position. In some implementations, a protruding end portion of the fastener main body 133 can be flanged, for example by the use of a die or any other suitable tool, to secure the fastener 134 in position.

Fig. 15-16B show an optional arrangement, in which the fastener head 135 is positioned radially inward, for example, abutting the clamp mid-portion 182^{a}, while the fastener main body 133 extends radially outward, toward and through the inner and outer struts 110^{a}b, 110^{a}a. In alternative arrangements, the fastener head 135 may be positioned radially outward, for example, abutting the outer strut 110^{a}a, while the fastener main body 133 extends radially inward, toward the valve longitudinal axis 10.

While the opening 190^{b} is shown in Figs. 16A-16B as having a uniform diameter, configured to accommodate the fastener main body 133 while the fastener head 135 may abut against the clamp mid-portion 182^{a}, in alternative implementations, the opening 190 may include a counter bore (not shown), similar to the counter-bore 122 shown for an aperture 118^{b} in Fig. 8, *mutatis mutandis.*

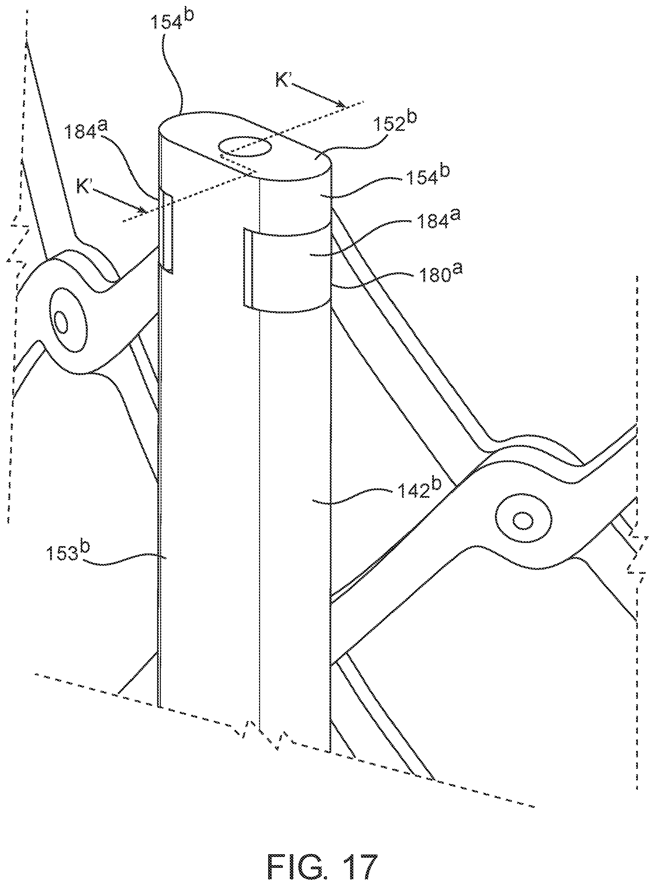

Fig. 17 shows a partial view in perspective of an outer member 142^{b} coupled to an outflow apex 130 via clamp 180^{a} of Fig. 15. Advantageously, the proposed configuration allows the outer member 142^{b} to snap into the clamp 180^{a} in a quick and simple manner.

While the clamp 180 is shown in Figs. 15-16B to be coupled to the struts 110 such that the clamp mid-portion 182 is positioned radially inward with respect to the inner strut 110b, in alternative arrangements, the clamp 180 may be coupled to the struts 110 such that the clamp mid-portion 182 is disposed between the inner and the outer struts 110b and 110a, respectively.

Advantageously, utilization of clamps (180) connected to junctions of the frame (106), and configured to snap over recessed portions of expansion and locking assemblies (140), allows for faster and more accurate attachment of expansion and locking assemblies (140) in either manual or automated production/assembly procedures.

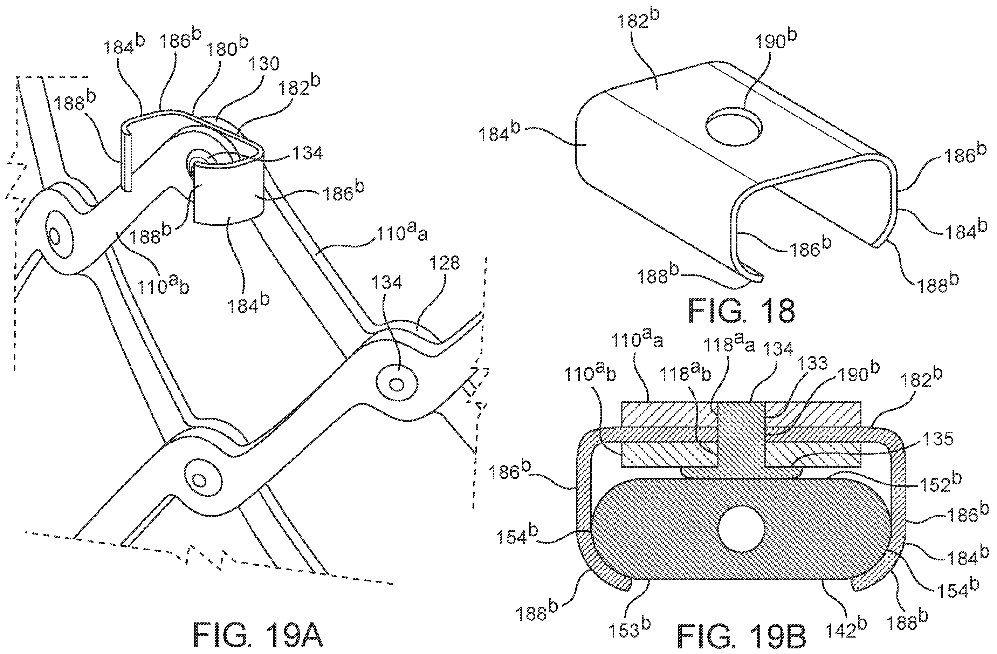

Fig. 18 shows a view in perspective of another type of clamp 180^{b}, which is similar to clamp 180^{a} except that each side arm 184^{b} includes an offsetting portion 186^{b} continuously extending from the clamp mid-portion 182^{b}, and an arcuate portion 188^{b} extending continuously from the offsetting portion 186^{b}, such that both opposite arcuate portions 188^{b} are bent toward each other. The offsetting portions 186^{b} are bent radially inward relative to the clamp mid-portion 182^{b}, and may optionally, but not necessarily, form straight portions of the side arms 184^{b} oriented perpendicularly to the clamp mid-portion 182^{b}.

Fig. 19A shows a view in perspective of a clamp 180^{b} attached to the struts 110 of a frame 106, which is similar to the view shown in Fig. 15, except that the clamp mid-portion 182^{b} is disposed between the inner and the outer struts 110^{a}b and 110^{a}a, respectively. While the clamp 180^{b} is shown in Fig. 19A coupled to an outflow apex 130, it will be clear that it may be coupled in the same manner to an inflow apex 132, or to any other non-apical junction 128.

Fig. 19B shows a cross-sectional view of the outflow apex 130 to which the clamp 180^{b} of Fig. 19A is attached, further illustrating the outer member 142^{b} snapped therein. As shown, placement of the clamp mid-portion 182^{b} between the outer strut 110^{a}a and the inner strut 110^{a}b may require elongation of the side arms 184^{b} relative to a configuration in which the clamp mid-portion 182^{a} is disposed radially inward with respect to the inner strut 110^{a}b, as shown in Figs. 15-16B, due to the additional thickness of the inner strut 110^{a}b. The purpose of the offsetting portions 186^{b} is to compensate for this additional inwardly oriented length, and to offset the arcuate portions 188^{b}, which are configured to press against the sidewalls 154^{b} of the outer member 142^{b}, further radially inward, away from the clamp mid-portion 182^{b}.

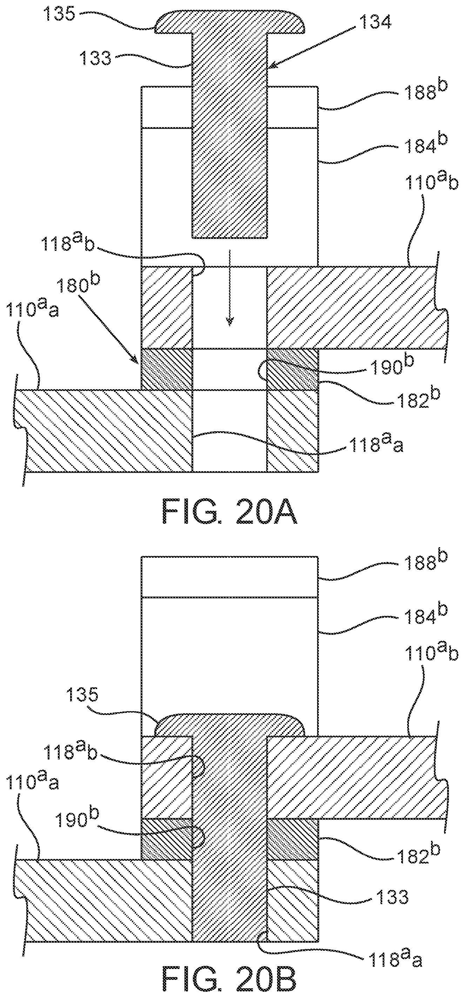

Figs. 20A and 20B show the clamp 180^{b}, along with an inner strut 110^{a}b and an outer strut 110^{a}a, as shown in Fig. 19A, before and after being engaged together, respectively. As shown in the illustrated configuration, both the inner strut 110^{a}b and the outer strut 110^{a}a are provided with an inner aperture 118^{a}b and an outer aperture 118^{a}a, respectively, at the junction to which the clamp 180^{b} is coupled. The opening 190^{b} of the clamp is aligned with, and disposed between, the inner aperture 118^{a}b and the outer aperture 118^{a}a, and may be preferably sized to have the same diameter such as that of the inner aperture 118^{a}b and the outer aperture 118^{a}a, matching the diameter of a fastener main body 133. As shown, a fastener 134 may be inserted through the inner aperture 118^{a}b, the opening 190^{b}, and the outer aperture 118^{a}a, to secure the clamp 180^{b} between the inner and outer struts 110^{a}b, 110^{a}a.

In some implementations, the length of the fastener main body 133 can be shorter than or equal to the combined thickness of the outer strut 110^{a}a, the clamp mid portion 182^{b}, and the inner strut 110^{a}b, when aligned with each other. In other implementations, the length of the fastener main body 133 can be longer than the combined thickness of the outer strut 110^{a}a, the clamp mid portion 182^{b}, and the inner strut 110^{a}b, when aligned with each other, enabling it to protrude beyond the outer strut 110^{a}a, such that an end cap or a nut (not shown) can be attached to the protruding end of the fastener main body 133 to secure the fastener 134 in position. In some implementations, a protruding end portion of the fastener main body 133 can be flanged, for example by the use of a die or any other suitable tool, to secure the fastener 134 in position.

Fig. 19A-20B show an optional arrangement, in which the fastener head 135 is positioned radially inward, for example, abutting the inner strut 110^{a}b, while the fastener main body 133 extends radially outward, toward and through the opening 190^{b} and the outer strut 110^{a}a. In alternative arrangements, the fastener head 135 may be positioned radially outward, for example, abutting the outer strut 110^{a}a, while the fastener main body 133 extends radially inward, toward the valve longitudinal axis 10.

While the exemplary arrangement of Figs. 19A-20B show an inner strut 110^{a}b of a type having apertures 118^{a}b with uniform diameters, other arrangements can include inner struts 110^{b}b provided with apertures 118^{b}b which include counter-bores 122 that can accommodate the fastener heads 135.

The cross-sectional view of Fig. 19B is taken across the region of the outer member 142^{b} that includes the outer member coupling recess 156^{b}, wherein the fastener head 135 is shown to be disposed between the inner strut 110^{a}b and the outer wall 152^{b} of the outer member 142^{b}, specifically at the region of the outer member coupling recess 156^{b}. In some implementations, the depth of the outer member coupling recess 156^{b} along the outer wall 152^{b} is substantially equal to, or even deeper than, the thickness of the fastener head 135. This will allow the fastener head 135 to be completely hidden within the outer member coupling recess 156^{b} in such a configuration.

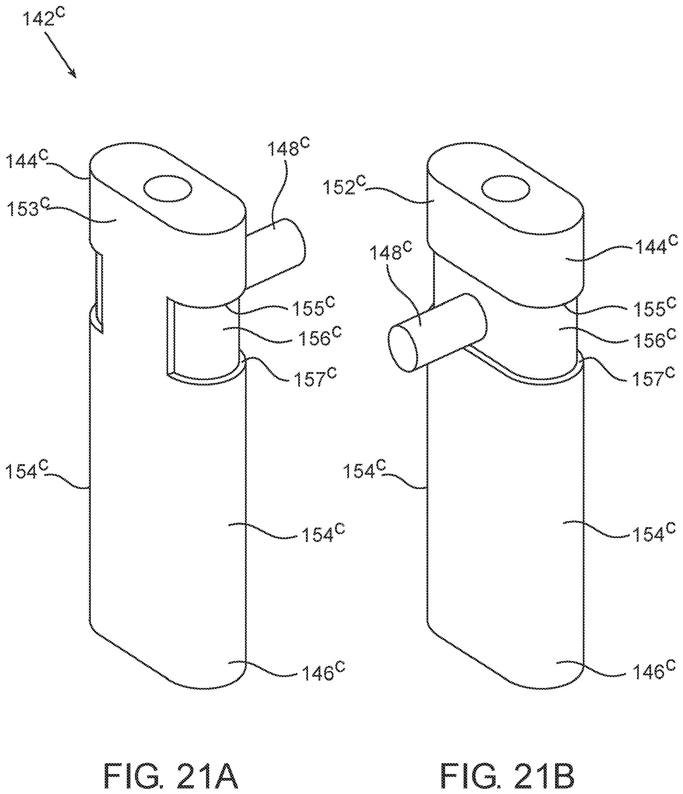

Figs. 21A-21B show another type of an outer member 142^{c}, which may be identical to outer member 142^{b} in all aspects, except that it further includes an outer member fastening extension 148^{c}, extending radially outward from the outer wall 152^{c} at the region of the outer member coupling recess 156^{c}.

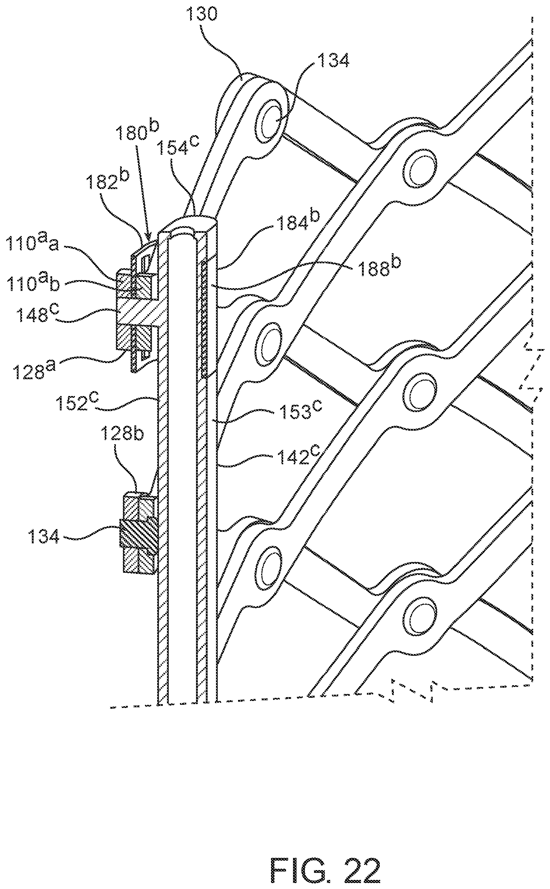

With reference to Fig. 22, showing an offset sectional view along a cutting plane k'-k' (which is shown, for example, in Fig. 17), an outer member 142^{c} can be coupled to a frame 106 by insertion the outer member fastening extensions 148^{c} through respective apertures 118 of the struts 110, and the opening 190 of the clamp 180. The inner and outer struts 110b, 110a at the remainder of the junctions 128 or apices 130, 132, which are not positioned at locations of expansion and locking assembly (140^{a}) attachment to the frame (106), may be joined together via fasteners 134. While outer member 142^{c} is shown in Fig. 22 coupled to a non-apical junction 128a, which may be distal to the outflow apices 130, it will be clear that it may be coupled in the same manner to an outflow apex 130, or to any other junction 128.

In some implementations, the length of the outer member fastening extensions 148^{c} can be shorter than or equal to the combined thickness of the outer strut 110^{a}a, the clamp mid portion 182^{b}, and the inner strut 110^{a}b, when aligned with each other. In other implementations, the length of the outer member fastening extensions 148^{c} can be longer than the combined thickness of the outer strut 110^{a}a, the clamp mid portion 182^{b}, and the inner strut 110^{a}b, when aligned with each other, enabling it to protrude beyond the outer strut 110^{a}a, such that an end cap or a nut (not shown) can be attached to the protruding end of the outer member fastening extensions 148^{c} to secure it in position. In some implementations, a protruding end portion of the outer member fastening extensions 148^{c} can be flanged (not shown), for example by the use of a die or any other suitable tool, to secure it in position.

While the exemplary arrangement illustrated in Fig. 22 shows the outer member 142^{c} coupled to a clamp 180^{b}, such that the outer member fastening extension 148^{c} extends through the opening 190^{b} of a clamp mid-portion 182^{b} disposed between an inner strut 110^{a}b and an outer strut 110^{a}a, in other arrangements, the outer member 142^{c} may be coupled to a clamp 180^{a}, such that the outer member fastening extension 148^{c} extends through the opening 190^{a} of a clamp mid-portion 182^{a} disposed positioned radially inward with respect to the inner strut 110^{a}b, *mutatis mutandis.*

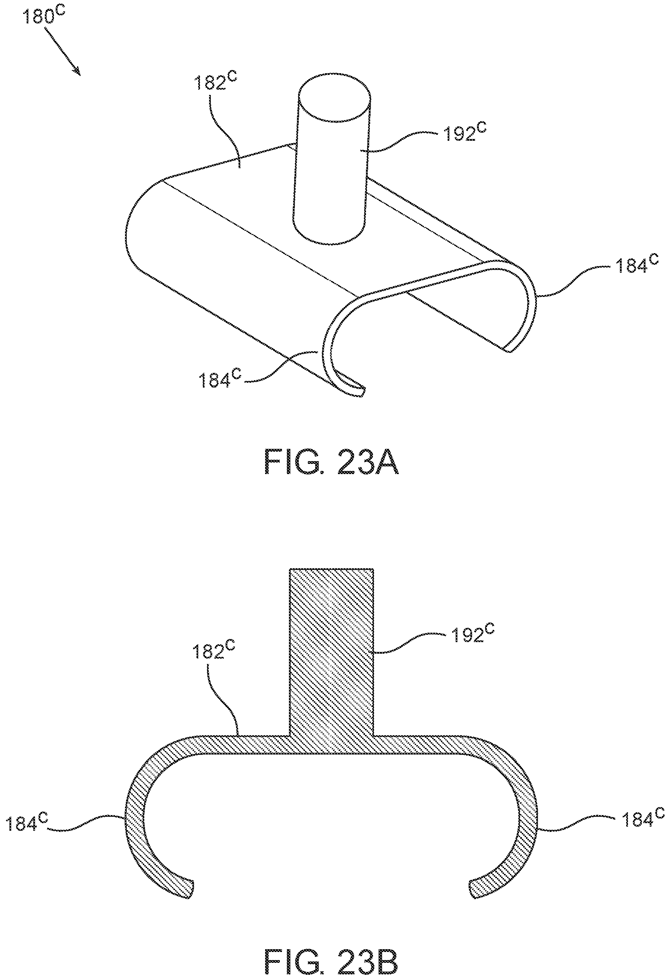

In some embodiments, a clamp 180 includes a clamp fastening extension 192, extending radially outward from the clamp mid-portion 182. Figs. 23A and 23B show a view in perspective and a cross-sectional view of another type of a clamp 180^{c}, which may be identical to clamp 180^{a}, except that clamp 180^{c} further includes a clamp fastening extension 192^{c} instead of an opening (190).

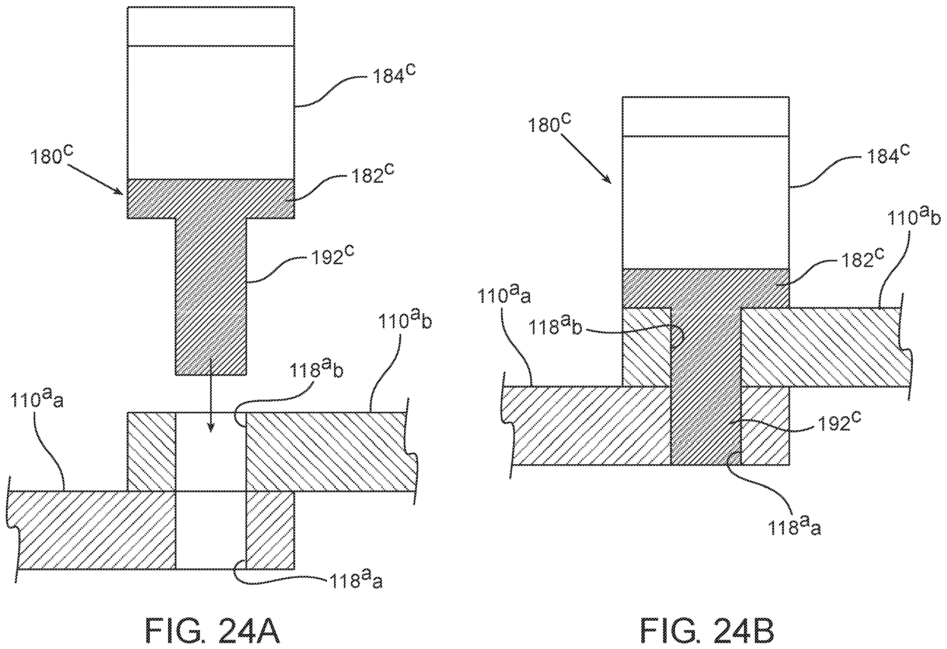

Figs. 24A and 24B show the clamp 180^{c}, along with an inner strut 110^{a}b and an outer strut 110^{a}a, before and after being engaged together, respectively. As shown in the illustrated configuration, both the inner strut 110^{a}b and the outer strut 110^{a}a are provided with an inner aperture 118^{a}b and an outer aperture 118^{a}a, respectively, at the junction to which the clamp 180^{c} is coupled. As shown, the clamp fastening extension 192^{c} may be inserted through the apertures 118^{a}b, 118^{a}a, to secure the clamp 180^{c} and both inner and outer struts 110^{a}b, 110^{a}a, together.

In some implementations, the length of the clamp fastening extension 192^{c} can be shorter than or equal to the combined thickness of the outer strut 110^{a}a and the inner strut 110^{a}b, when aligned with each other. In other implementations, the length of the clamp fastening extension 192^{c} can be longer than the combined thickness of the outer strut 110^{a}a and the inner strut 110^{a}b, when aligned with each other, enabling it to protrude beyond the struts 110, such that an end cap or a nut (not shown) can be attached to the protruding end of the clamp fastening extension 192^{c} to secure the clamp 180^{c} in position. In some implementations, a protruding end portion of the clamp fastening extension 192^{c} can be flanged (not shown), for example by the use of a die or any other suitable tool, to secure clamp 180^{c} in position.

While the clamp 180^{c} illustrated in Figs. 23A-23B is shown with arched side arms 184^{c} of the type illustrated in Figs. 13A-13B for a clamp 180^{a}, it will be clear that this is merely shown for illustrative purpose, and that a clamp 180^{c} having a clamp fastening extension 192^{c}, may be similarly provided with side arms of the type shown for side arms 184^{b} illustrated in Fig. 18, i.e., provided with offsetting portions 186 and arcuate portions 188, *mutatis mutandis.* Such a configuration may be useful for placement of a clamp 180^{c} having a clamp fastening extension 192^{c}, between the inner strut 110^{a}b and the outer strut 110^{a}a, in an arrangement similar to that shown in Figs. 19A-20B, *mutatis mutandis.* In such a configuration (not shown separately), the length of the clamp fastening extension 192^{c} can be shorter than or equal to the thickness of the outer strut 110^{a}a. Alternatively, the length of the clamp fastening extension 192^{c} can be longer than the thickness of the outer strut 110^{a}a, enabling it to protrude beyond the outer strut 110^{a}a, such that an end cap or a nut (not shown) can be attached to the protruding end of the clamp fastening extension 192^{c}. In some implementations, a protruding end portion of the clamp fastening extension 192^{c} can be flanged (not shown), for example by the use of a die or any other suitable tool.

In some implementations, an inner member 160 can include an inner member coupling recess 172. In some implementations, the inner member distal end portion 164 comprises the inner member coupling recess 172. The inner member distal end portion 164 comprises an inner member distal outer wall 166 configured to face the frame (106), an opposite inner member distal inner wall configured to face the valve longitudinal axis 10, and two inner member distal sidewalls 168. inner member coupling recess 172 spans across the inner member distal sidewalls 168, and optionally along at least a portion of the inner member distal inner wall. Optionally, but not necessarily, the inner member coupling recess 172 may also span across the inner member distal outer wall 166.

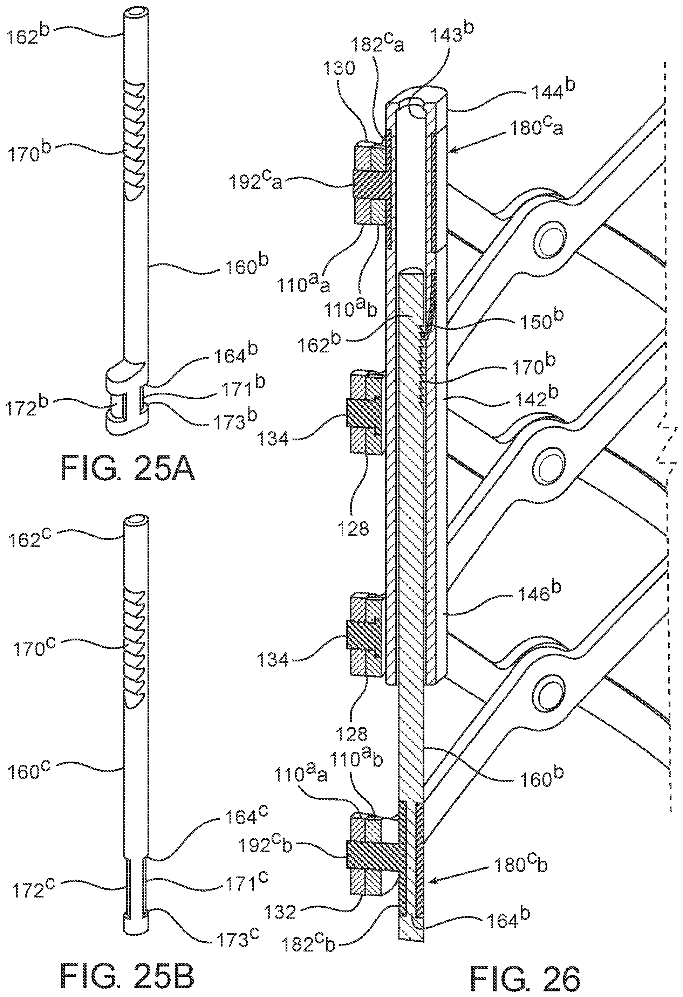

Fig. 25A and 25B show various exemplary types of inner members 160 provided with an inner member coupling recess 172. Fig. 25A shows an exemplary embodiment of an inner member 160^{b}, comprising an inner member coupling recess 172^{b} configured to accommodate at least a portion of a clamp 180 therein. The inner member coupling recess 172^{b} shown in Fig. 25A is shaped so as to closely match the shape of the clamp 180^{a} shown in Fig. 13A-13B, for example. The inner member distal end portion 164^{b} can be formed as a wider portion, relative to the remaining portion of the inner member 160^{b} extending proximally therefrom. The inner member distal outer wall 166^{b} may be provided as a generally flat wall, while the inner member distal sidewalls 168^{b} may be rounded. The inner member distal inner wall may be flat and parallel to the inner member distal outer wall 166^{b}.

In some implementations, the height of the inner member coupling recess 172, defined as the distance between a proximal edge and a recess distal edge (173) thereof, is substantially equal to the longitudinal length of the clamp 180. That is to say, the height of the inner member coupling recess 172 is not greater than 110% of the longitudinal length of the clamp 180.

In some implementations, as shown in Fig. 15A, the inner member coupling recess 172^{b} spans across the inner member distal outer wall 166^{b}, configured to accommodate the clamp mid-portion 182^{a}, and across the inner member distal sidewalls 168^{b}, configured to accommodate the side arms 184^{a}.

Fig. 15B shows another type of an inner member 160^{c}, which may be identical to the inner member 160^{b}, except that the inner member distal end portion 164^{c} is not formed as a wider portion as is the case with the inner member distal end portion 164^{b}, but may be shaped with the same dimensions, and potentially the same overall round cross-sectional shape, as that of the remainder of the inner member 160^{c}. In this case, the inner member coupling recess 172^{b} may be C-shaped along a portion of the inner member distal end portion 164^{c}.

An inner member 160 having an inner member coupling recess 172, such as either inner member 160^{b} or inner member 160^{c}, may be used in combination with any of the types of a clamp 180, such as clamp 180^{a}, clamp 180^{b}, or clamp 180^{c}, for attachment to a junction 128 or an apex 130, 132 of a frame (106) according to any of the arrangements described herein above for outer member 142 having outer member coupling recesses 156, such as outer member 142^{b} or outer member 142^{c}, *mutatis mutandis.*

With reference to Fig. 26, an expansion and locking assembly 140 can be coupled to the struts 110 of a frame 106 at two axially spaced junctions 128 (including apices 130, 132), via two clamps 180, such as a first clamp 180a utilized for coupling an outer member 142 to the frame 106 at a first location, and a second clamp 180b utilized to couple an inner member 160 to the frame 106 at a second location. Fig. 26 shows an expansion and locking assembly 140 comprising an outer member 142^{b} coupled to an outflow apex 130 via a first clamp 180^{c}a, and an inner member 160^{b} coupled to an inflow apex 132 via a second clamp 180^{c}b. The first clamp 180^{c}a is shown with a clamp fastening extension 192^{c}a extending through apertures 118 of an inner strut 110^{a}b and an outer strut 110^{a}b. Similarly, the second clamp 180^{c}b is shown with a clamp fastening extension 192^{c}b also extending through apertures 118 of an inner strut 110^{a}b and an outer strut 110^{a}b.

While the first location to which the first clamp 180^{c}a is attached is shown as an outflow apex 130, and the second location to which the second clamp 180^{c}b is attached is shown as the inflow apex 132, it will be clear that in other arrangements, the first location may be the inflow apex 132 or any other non-apical junction 128, and that the second location may be the outflow junction 130 or any other non-apical junction 128.

While the first clamp 180^{c}a is illustrated in the exemplary embodiments with an integral clamp fastening extension 192^{c}a, and the second clamp 180^{c}b is illustrated with an integral clamp fastening extension 192^{c}b, it will be clear that this is just for illustrative purpose, and that any of the first and second clamps can be of any other type of clamp 180 disclosed throughout the current specification, including, but not limited to, clamps 180^{a} having a clamp mid-portion 182^{a} disposed radially inward with respect to the inner struts 110^{a}b, or clamps 180^{b} having a mid-portion 182^{b} disposed between the outer struts 110^{a}a and the inner struts 110^{a}b, *mutatis mutandis.*

In some implementations, an inner member 160 provided with an inner member coupling recess 172, such as illustrated in Figs. 25A or 25B, can further comprise an inner member fastening extension 176, extending radially outward from the inner member coupling recess 172, in a similar manner shown for outer member 142^{c} in Figs. 21A-21B, and utilized for coupling to inner and outer struts 110^{a}b, 110^{a}a, according to any of the arrangements described hereinabove in relation to outer member 142^{c}, *mutatis mutandis.*

In various implementations, clamps 180 provided with openings 190 can be coupled to the struts 110 either via fasteners 134, or alternatively used in combination with outer members 142^{c} having outer member fastening extensions 148^{c} extending radially outward from their outer member coupling recesses 156^{c}, and/or inner members 160 having inner member fastening extensions 176 extending radially outward from their inner member coupling recess 172.

While Fig. 26 shows an exemplary arrangement of both first and second clamps 180^{c}a, 180^{c}b, being of the same type 180^{c}, in alternative arrangements, the first clamps 180a can be of a different type than that of the second clamps 180b. Moreover, regardless of whether the first and second clamps 180a, 180b are of the same type or of different types, each of the first and second clamps 180a, 180b, can have different dimensions, configured to accommodate the outer member 142 and the inner member 160 attached thereto. For example, in most cases, the circumference of the second clamp 180b will be smaller than that of the first clamp 180a, as the second clamp 180b is dimensioned to snap over an inner member 160, which may be provided with a smaller circumference than that of the outer member 142, specifically in expansion and locking assemblies 140 in which the inner member 160 is at least partially extendable through a lumen 143 of the outer member 142. Nevertheless, in specific implementations, the inner member 160 can be provided with a wider distal end portion 164, with dimensions that will require a second clamp 180b with a circumference equal to, or even larger than, that of a first clamp 180.

Some types of prosthetic valves (100) can include a plurality of commissure support elements configured as commissure clasps or clips (not shown). Such commissure clips can be mounted on an expansion and locking mechanism (14), or mounted to struts (110) of the frame (106). Further details of commissure clamps and techniques for mounting the commissures 138 to a frame (106) can be found in U.S. Patent Application Publication No. 2018/0325665.

According to some embodiments, the clamp 180 can further comprise clamp axial arms 194 configured to serve as commissure attachment portions, enabling the clamp 180 to serve not only as a means for attaching the expansion and locking assembly (140) to the frame (106), but also as a commissure attachment means.

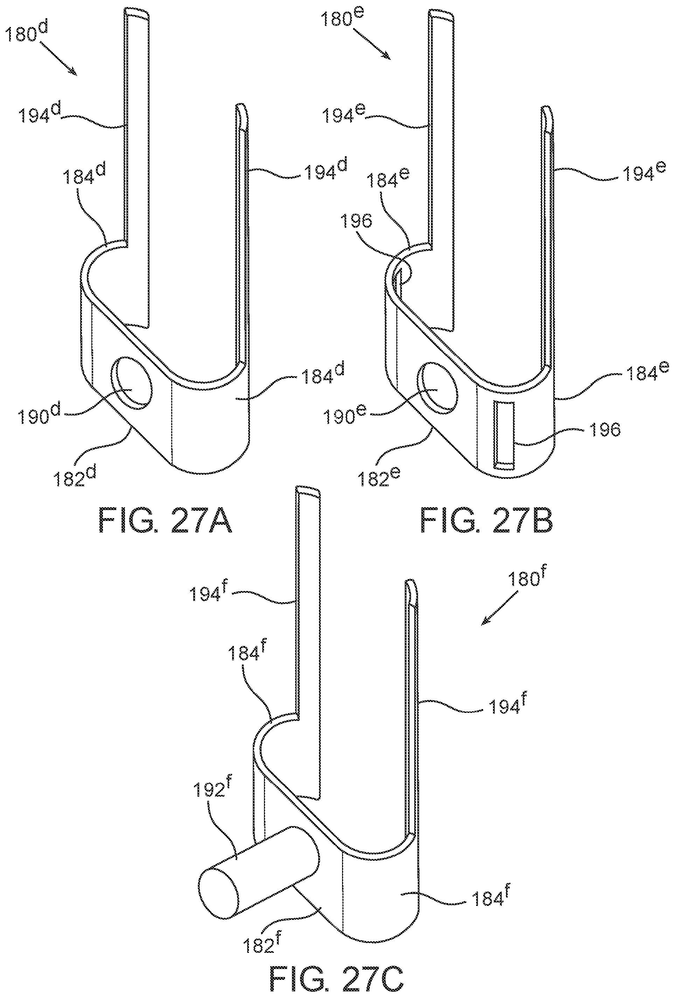

Figs. 27A-27C show additional types of clamps 180, further comprising clamp axial arms 194, axially extending from the free ends 185 of the side arms 184. Fig. 27A shows an exemplary embodiment of a clamp 180^{d}, which may be identical to clamp 180^{a}, except that it further includes a clamp axial arm 194^{d} extending axially from the free end 185 of each of the side arms 184^{d}. The clamp axial arms 194^{d} are opposite to the clamp mid-portion 182^{d}, and extend to a height which is beyond the height of the clamp mid-portion 182^{d} and the remainder portion of the side arms 184^{d}. The clamp 180^{d} can be attached to the struts 110 of the frame (106) according to any of the arrangements described hereinabove for other clamps (180) embodiments, including, but not limited to, positioning the clamp 180^{d} radially inward, with respect to the inner strut 110^{a}b, and attaching it to both the inner and outer struts 110^{a}b, 110^{a}a via fastener 134.

While the shape of the exemplary clamp 180^{d} illustrated in Fig. 27A is shown with arched side arms 184^{c} of the type illustrated in Figs. 13A-13B for a clamp 180^{a}, it will be clear that this is merely shown for illustrative purpose, and that a clamp 180^{d} having clamp axial arms 194^{d}, may be similarly provided with side arms of the type shown for side arms 184^{b} illustrated in Fig. 18, i.e., provided with offsetting portions 186 and arcuate portions 188, *mutatis mutandis.* Such a configuration may be useful for placement of a clamp 180^{d} having a clamp fastening extension 192^{c}, between the inner strut 110^{a}b and the outer strut 110^{a}a, in an arrangement similar to that shown in Figs. 19A-20B, *mutatis mutandis.*

Fig. 27B shows another exemplary embodiment of a clamp 180^{e}, which is identical to any of the embodiments described in relation to clamp 180^{d}, except that clamp 180^{e} further comprises slits 196, which can be designed as generally axially oriented slits 196 extending through the thickness of side arms 184^{e}, configured to allow at least one strut 110 to extend therethrough. This may be useful for configurations in which the clamp mid-portion 182^{e} is positioned between an outer strut 110^{a}a and an inner strut 110^{a}b. In such cases, the movement of the inner strut 110^{a}b may be interrupted by the side arms 184^{e}. Extension of the inner strut 110^{a}b through at least one slit 196 may allow it to move and pivot about the junction (128) to which the clamp 180^{e} is attached, without interference. In some implementations, the width of the slit 196 may be at least equal to, or higher than, the thickness of the struts 110, such as the thickness of the inner strut 110^{a}b, allowing it to extend therethrough. In some implementation, the axial length of the slit 196 is larger than the width of a linear segment 112 of a strut 110, so as to allow the strut 110 to pivot about the junction (128) to which the clamp 180^{e} is attached, between a crimped configuration and a fully expanded configuration of the valve (100).

While clamp 180^{e} is illustrated in Fig. 27B with two opposing slits 196 comprised in both side arms 184^{e}, in some applications, a single slit 196 comprised within only one of the side arms 184^{e} may suffice, for example if the clamp 180^{e} is attached to an apex, such as an outflow apex (130), such that the inner strut 110^{a}b extends only from one side of the clamp 180^{e}.

While the shape of the exemplary clamp 180^{e} illustrated in Fig. 27B is shown with arched side arms 184^{e} of the type illustrated in Figs. 13A-13B for a clamp 180^{a}, it will be clear that this is merely shown for illustrative purpose, and that a clamp 180° having slits 196, may be similarly provided with side arms of the type shown for side arms 184^{b} illustrated in Fig. 18, i.e., provided with offsetting portions 186 and arcuate portions 188, *mutatis mutandis.* Moreover, it will be understood that slits 196 of the type described and illustrated for 180° can be combined into any of the other types of clamps (180) of the current disclosure, such as clamp 180^{a}, clamp 180^{b}, or clamp 180^{c}.

Fig. 27C shows another exemplary embodiment of a clamp 180^{f}, which is identical to any of the embodiments described in relation to clamp 180^{d}, except that clamp 180^{f} comprises a clamp fastening extension 192^{f} instead of an opening (190^{d}). The clamp 180^{f} can be attached to the struts 110 of the frame (106) according to any of the arrangements described hereinabove for other clamp 180^{c} with respect to Figs. 23A-24B.

While the shape of the exemplary clamp 180^{f} illustrated in Fig. 27C is shown with arched side arms 184^{f} of the type illustrated in Figs. 13A-13B for a clamp 180^{a}, it will be clear that this is merely shown for illustrative purpose, and that a clamp 180^{f} having clamp axial arms 194^{f} and a clamp fastening extension 192^{f}, may be similarly provided with side arms of the type shown for side arms 184^{b} illustrated in Fig. 18, i.e., provided with offsetting portions 186 and arcuate portions 188, *mutatis mutandis.* While not separately illustrated, it will be clear that clamp 180^{f} can further comprises slits 196 of the type illustrated in Fig. 27B.

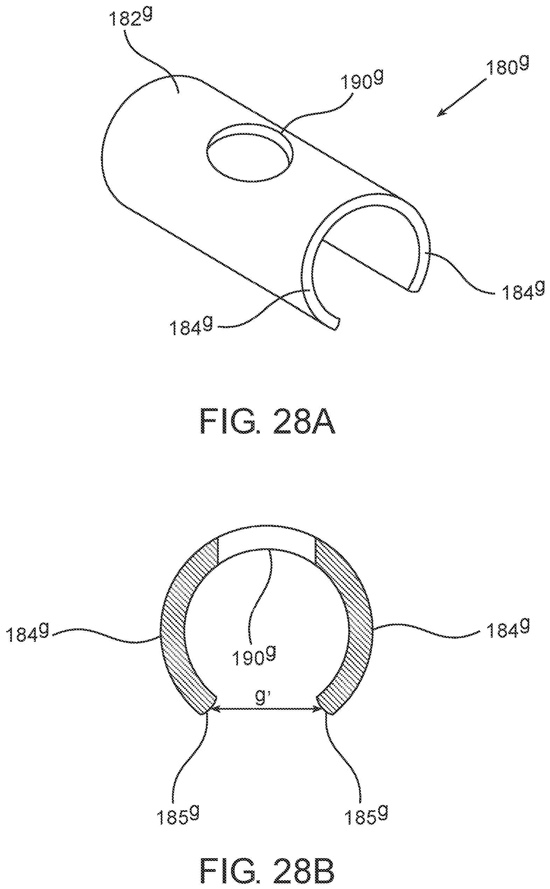

Figs. 28A-28B show a view in perspective and a cross-sectional view of another type of a clamp 180^{g}. Clamp 180^{g} differs from other types of clamps (180) disclosed hereinabove, in that it has a generally circular, C-shaped cross-sectional contour, allowing it to retain circularly shaped components, such as a circularly shaped outer member (142) or a circularly shaped inner member (160). Thus, the clamp mid-portion 182^{g} and the side arms 184^{g} constitute a continuous structure, that may be formed, for example, by cutting a tubular member to form a gap g' between the free ends 185^{g} of the side arms 184^{g}. A C-shaped clamp 180^{g} can be used, for example, to clamp an inner member 160^{c} of the type shown in Fig. 25B, which is provided with a circularly shaped inner member distal end portion 164^{c}, having a complementary C-shaped inner member coupling recess 172^{c}.

While the C-shaped clamp 180^{g} is illustrated in Figs. 28A-28B with an opening 190^{g}, it will be clear that it may be similarly provided with a clamp fastening extension (192). The C-shaped shaped clamp 180^{g} may be coupled to inner and outer struts 110^{a}b, 110^{a}a, according to any of the arrangements described hereinabove for any of the other types of clamps (180). While not separately illustrated, it will be clear that C-shaped shaped clamp 180^{g} can further comprises slits 196 of the type illustrated in Fig. 27B.

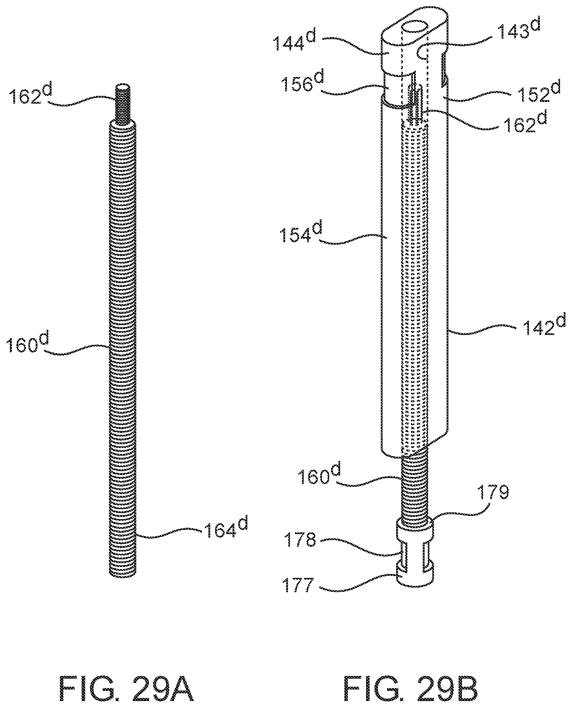

With reference to Figs. 29A-29B, other types of expansion and locking assemblies (140) can include additional components attachable to the frame (106), such as distal nuts 177 configured to threadedly engage with a distal threaded portion of the inner member (160). Fig. 29A shows an exemplary embodiment of an inner member 160^{d} which may be provided as an elongated rod having an external thread. The inner member 160^{d} may include, in specific implementations, a shorter and narrower threaded portion along the inner member proximal end portion 162^{d}, configured to releasable engage with an actuation assembly. While the exemplary inner member 160^{d} is illustrated in Fig. 29A with an external thread spanning along its entire length, in other implementations, the external thread may be disposed only over the inner member distal end portion 164^{c} (embodiment not shown).

As shown in Fig. 29B, the inner member 160^{d} may at least partially extend through the lumen 143^{d} of an outer member 142^{d}, having its inner member distal end portion 164^{c} threaded into a distal nut 177. The distal nut 177 is open ended at its proximal opening 179 (and potentially, open ended at its opposite distal end as well), and includes an internal thread configured to engage with the external thread of the inner member distal end portion 164^{c}. Additional components of this type of an expansion and locking assembly may include a locking nut (not shown) disposed within the outer member 142^{d}, which may be configured to lock the frame (106) into a particularly radially expanded state. Further details regarding the structure and mechanism of operation of such expansion and locking assemblies are further described in U.S. Publication Nos. 2018/0153689.

The outer member 142^{d} comprises an outer member coupling recess 156^{d}, and is illustrated in Fig. 29B to have an external shape similar to that of outer member 142^{b}, yet other external shapes are contemplated.

The distal nut 177 comprises a nut coupling recess 178, which can be shaped to receive a clamp (180) of any of the clamp embodiments disclosed hereinabove. The nut coupling recess 178 can be similar to any of the embodiments described hereinabove for any of the outer member coupling recess (156) or the inner member coupling recess (172), *mutatis mutandis.* While the distal nut 177 is shown to have an external circular or ring-shaped contour in Fig. 29B, it is to be understood that outer external shapes are contemplated, such as rectangular shapes with rounded sidewalls, D-shaped members, and the like.

With reference to Fig. 30A, the outer member 142^{d} can be coupled to the struts 110 of a frame 106 according to any of the embodiments described hereinabove for coupling any type of an outer member (142) to the frame (106). The outer member coupling recess 156^{d} can be shaped according to any of the embodiments described hereinabove for outer member coupling recess 156^{b}, and may be coupled to any of the clamps (180), and according to any coupling arrangements, described throughout the current specification. In some implementations, the outer member 142^{d} can further comprise an outer member fastening extension (148) extending radially outward from the outer member coupling recess 156^{d}, designed according to any of the embodiments described for outer member 142^{c}, and coupled to the frame (106) according to any arrangement disclosed hereinabove for coupling an outer member 142^{c} to the frame (106), *mutatis mutandis.*

Fig. 30A shows an expansion and locking assembly (140) of the type shown in Fig. 29B, having the outer member 142^{d} coupled to the frame 106 via a first clamp 180^{d}a, and a distal nut 177 coupled to the frame 106 via a second clamp 180^{g}b, exemplifying a configuration in which two different types of clamps (180) are utilized. In the specific illustrated example, a C-shaped second clamp 180^{g}b is shown, utilized for clamping a ring-shaped distal nut 177. It will be understood that this configuration is non-binding, and that other types of clamps (180) can be utilized with different variations of the distal nut (177).

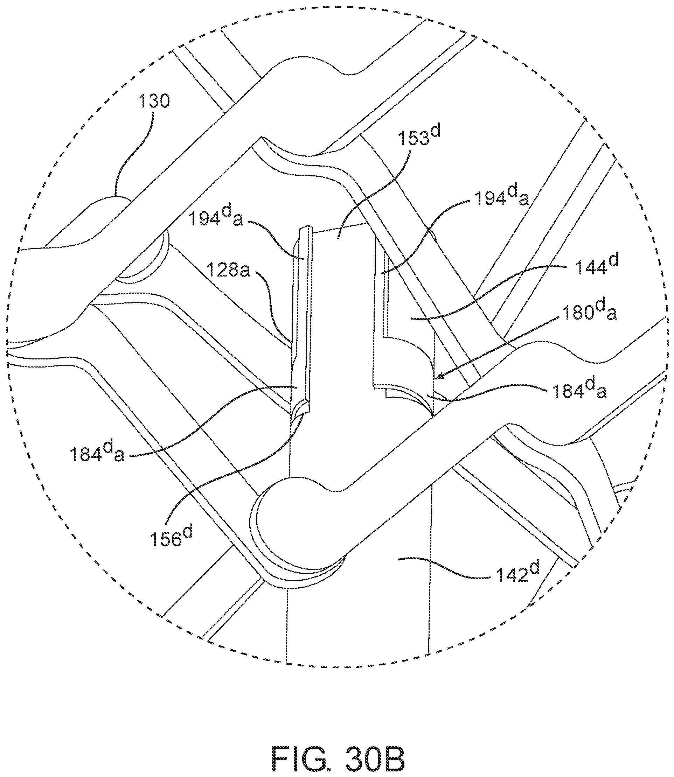

Fig. 30B is an enlarged view of region 30B from Fig. 30A, showing a first clamp 180^{d}a provided with clamp axial arms 194^{d}a, utilized for clamping the outer member 142^{d} to the frame. It is to be understood that the illustrated configuration is merely a non-binding example, and that the outer member 142^{d} can be attached to the frame 106 via other types of clamps (180), in the same manner that the clamp 180^{d}a can be used to attached other types of outer member (142) to the frame (106). Specifically, Fig. 30B may be illustrative not only of an outer member 142^{d}, but of any of the outer members 142^{b} and 142^{c} as well. Similarly, Fig. 30B may be illustrative not only of a clamp 180^{d} provided with axial arms 194^{d}, but of clamps 180^{e} or 180^{f} that may be also provided with the same set of axial arms (194) for commissure attachment.

As shown, a clamp 180^{d}, 180^{e}, 180^{f}, provided with clamp axial arms 194, is designed such that the clamp axial arms 194 are offset radially inward with respect to the outer member 142, and more specifically, offset radially inward with respect to the inner wall 153 of the outer member 142. In some implementations, the extent of the offset is at least equal to, or may be larger than, the thickness of the tabs (137) of leaflets (136), allowing the tabs (137) to extend through the gap formed between the clamp axial arms 194 and the inner wall 153 of the outer member 142.

In some implementations, as shown in Fig. 30B, the clamp axial arms 194 are oriented in a proximal direction, with respect to the outer member coupling recess 156. In some implementations, the clamp axial arms 194 may extend proximally beyond the outflow end 103 of the frame (106). In other implementations, the proximal ends of the clamp axial arms 194 are configured not to extend proximally beyond the outflow end 103 of the frame (106).

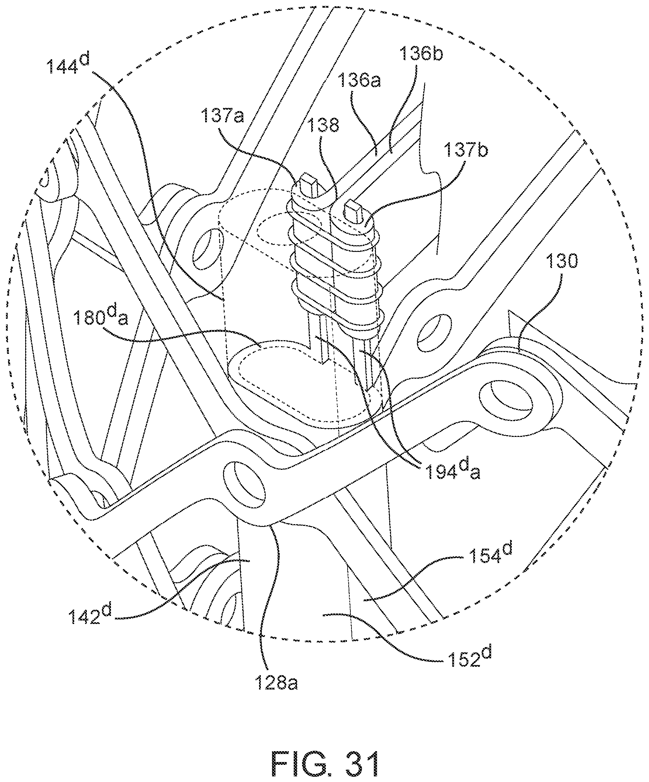

With reference to Fig. 31, the tabs 137 of adjacent leaflets 136 can be inserted through the gap g' formed between the clamp axial arms 194, and may be wrapped around the clamp axial arms 194, extending through the gap formed between the clamp axial arms 194 and the outer member 142. A suture may be used for attachment of the tabs 137 to the clamp axial arms 194, and potentially to each other, to form a commissure 138. Additional elements, such as wedges and cloth strips or fabrics (not shown) may be added during the formation of commissure (138). Further details regarding various attachments techniques and mechanisms for formation of commissures are disclosed in U.S. Publication No. 2018/0325665; U.S. Publication No. 2019/0105153; U.S. Application No. 62/869,948; U.S. Application No. 62/813,643; and PCT Application No. PCT/US2019/61392.

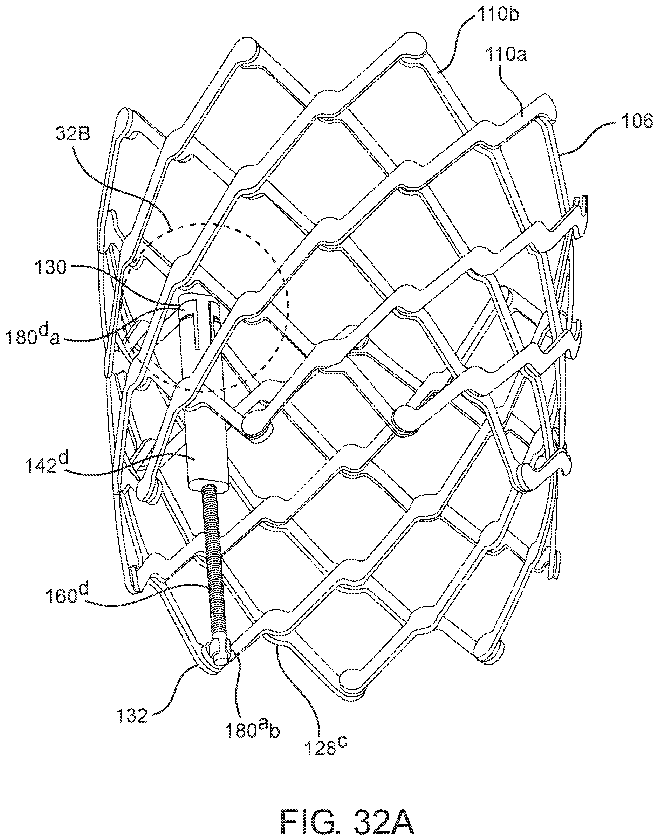

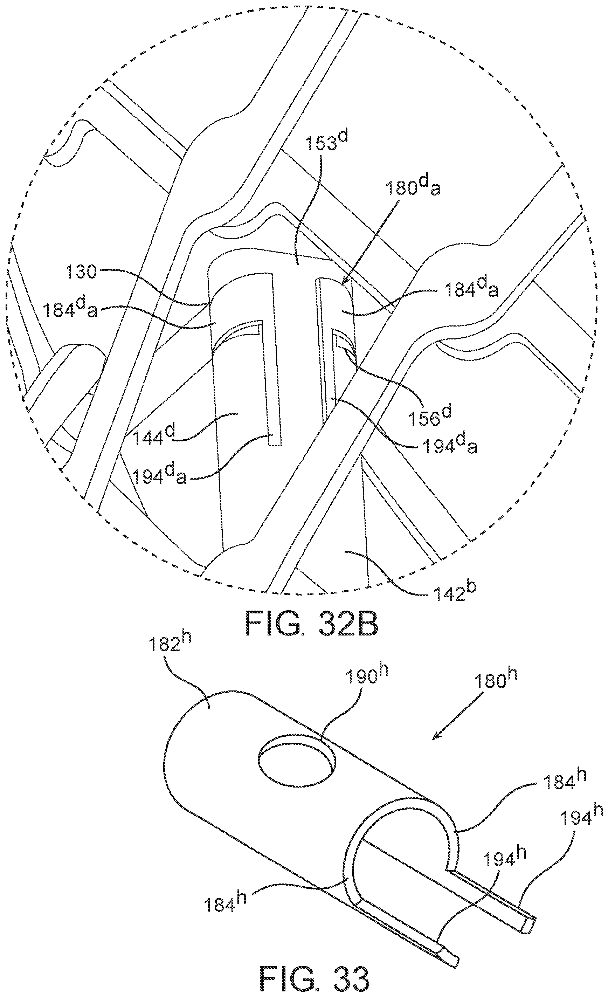

Figs. 32A-32B show another attachment configuration, which may be similar to the embodiments described in conjunction with Figs. 31A-31B above, except that the clamp axial arms 194, in the illustrated exemplary embodiment, are oriented in a distal direction with respect to the outer member coupling recess 156. While not explicitly illustrated, the clamp axial arms 194 may further include lateral extensions, extending from their distal ends, configured to support the commissure (138) and prevent the tabs (137) from slipping away from the clamp axial arms 194 in such configurations.

Fig. 33 shows another exemplary type of a clamp 180^{h}, which combines the C-shaped configuration of the clamp 180^{g}, with clamp axial arms 194^{h} which are similar to the clamp axial arms 194^{d} of clamp 180^{d}. While clamp 180^{h} is illustrated in Fig. 33 to have an opening 190^{h}, it will be clear that in alternative implementations, clamp 180^{h} may include a clamp fastening extension, similar to clamp fastening extension 190^{f} of clamp 180^{f}. Moreover, while not explicitly illustrated, it is to be understood that in some implementations, clamp 180^{h} can further include slits 196, similar to those of clamp 180^{e}.

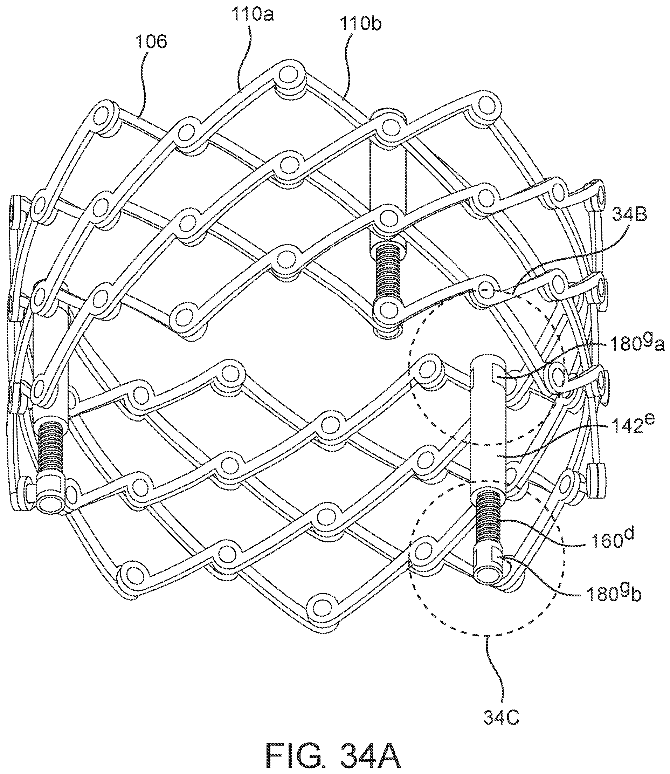

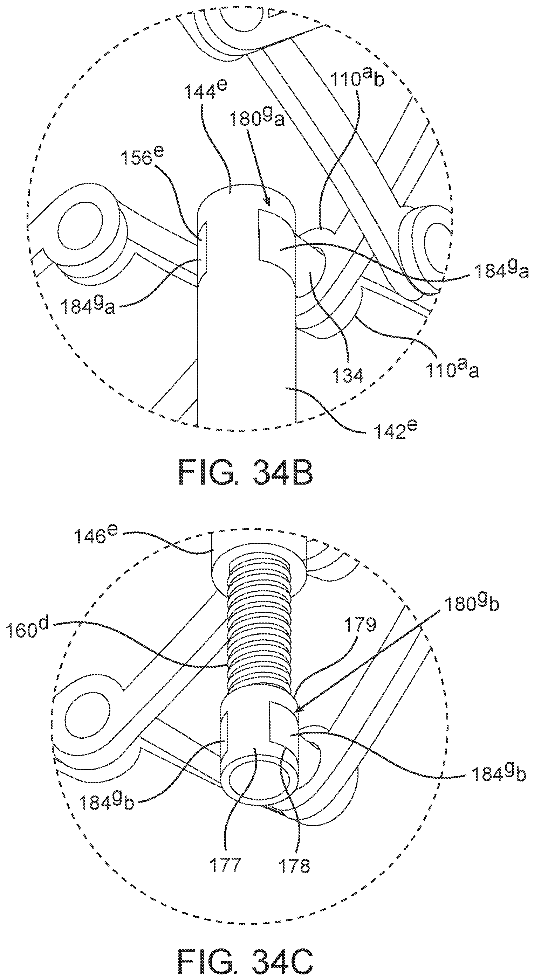

With reference to Figs. 34A-34C, an expansion and locking assembly (140) can include, in some embodiments, a tubular outer member 142^{e}, having a corresponding C-shaped outer member coupling recess 156^{e}. A C-shaped first clamp 180^{g}a can be utilized for clamping a tubular outer member 142°, while a C-shaped second clamp 180^{g}b can be utilized for clamping a ring-shaped distal nut 177. The dimensions of the C-shaped first clamp 180^{g}a and the C-shaped second clamp 180^{g}b can differ from each other, or can be similar to each other, depending on the external dimension of the outer member coupling recess 156° and the nut coupling recess 178, respectively. While a C-shaped member 180^{g} is shown to clamp the tubular outer member 142° in the illustrated examples, in other arrangements, a C-shaped member 180^{h} having clamp axial arms 194^{h} may be utilized, to serve as a coupling means for commissures (138) in a similar manner to that described with respect to Figs. 30A-32B, *mutatis mutandis.*

It is appreciated that certain features of the invention, which are, for clarity, described in the context of separate embodiments, may also be provided in combination in a single embodiment. No feature described in the context of an embodiment is to be considered an essential feature of that embodiment, unless explicitly specified as such.

In view of the many possible embodiments to which the principles of the disclosure may be applied, it should be recognized that the illustrated embodiments are only preferred examples and should not be taken as limiting the scope. Rather, the scope is defined by the following claims.

## Claims

1. A prosthetic valve (100), comprising:
a frame (106) movable between a radially compressed and a radially expanded configuration;
at least one clamp (180) coupled to the frame, comprising: a clamp mid-portion (182) comprising an opening (190) extending through its thickness, and a couple of opposing side arms (184) continuously extending from the clamp mid-portion; and
at least one expansion and locking mechanism (140), comprising:
an outer member (142), coupled to the frame at a first location; and
an inner member (160), coupled to the frame at a second location spaced apart from the first location, the inner member extending at least partially into the outer member;
wherein movement of the inner member in a first direction, relative to the outer member, causes the frame to foreshorten axially and expand radially;
wherein the side arms are resiliently expandable away from each other, and are inwardly biased toward each other in the absence of an expanding force applied thereto; and
wherein at least one of: the outer member and/or the inner member, is coupled to the frame via the at least one clamp.

2. The prosthetic valve of claim 1, wherein the frame comprises a plurality of inner struts (110b) and a plurality of outer struts (110a), pivotably interconnected at junctions (128), and wherein the at least one clamp is attached to an inner strut and an outer strut at a junction formed therebetween.

3. The prosthetic valve of any one of claims 1 or 2, wherein each of the side arms comprises an offsetting portion (186) continuously extending from the clamp mid-portion, and an arcuate portion (188) continuously extending from the offsetting portion.

4. The prosthetic valve of any one of claims 1 to 3, wherein the at least one clamp further comprises clamp axial arms (194), extending axially from the side arms; optionally:
a.) wherein the clamp axial arms are offset radially inward with respect to the outer member, and/or
b.) further comprising a plurality of leaflets (136), each leaflet comprising a pair of oppositely-directed tabs (137), wherein the tabs of each two adjacent leaflets are coupled to the clamp axial arms, thereby forming a commissure (138).

5. The prosthetic valve of any preceding claim, wherein the clamp further comprises slits (196) extending through the thickness of the side arms.

6. The prosthetic valve of any preceding claim, wherein the at least one clamp (180) comprises a first clamp (180a) coupled to the frame at the first location, and a second clamp (180b) coupled to the frame at the second location, wherein the outer member is coupled to the first clamp, and wherein the inner member is coupled to the second clamp.

7. A prosthetic valve (100), comprising:
a frame (106) movable between a radially compressed and a radially expanded configuration;
at least one clamp (180) coupled to the frame, comprising: a clamp mid-portion (182) comprising a clamp fastening extension (192) extending radially outward therefrom, and a couple of opposing side arms (184) continuously extending from the clamp mid-portion; and
at least one expansion and locking mechanism (140), comprising:
an outer member (142), coupled to the frame at a first location; and
an inner member (160), coupled to the frame at a second location spaced apart from the first location, the inner member extending at least partially into the outer member;
wherein movement of the inner member in a first direction, relative to the outer member, causes the frame to foreshorten axially and expand radially;
wherein the side arms are resiliently expandable away from each other, and are inwardly biased toward each other in the absence of an expanding force applied thereto; and
wherein at least one of: the outer member and/or the inner member, is coupled to the frame via the at least one clamp.

8. The prosthetic valve of claim 7, wherein the frame comprises a plurality of inner struts (110b) and a plurality of outer struts (110a), pivotably interconnected at junctions (128), and wherein the at least one clamp is attached to an inner strut and an outer strut at a junction formed therebetween.

9. The prosthetic valve of any one of claims 7 or 8, wherein each of the side arms comprises an offsetting portion (186) continuously extending from the clamp mid-portion, and an arcuate portion (188) continuously extending from the offsetting portion.

10. The prosthetic valve of any one of claims 7 to 9, wherein the at least one clamp further comprises clamp axial arms (194), extending axially from the side arms; optionally:
a.) wherein the clamp axial arms are offset radially inward with respect to the outer member, and/or
b.) further comprising a plurality of leaflets (136), each leaflet comprising a pair of oppositely-directed tabs (137), wherein the tabs of each two adjacent leaflets are coupled to the clamp axial arms, thereby forming a commissure (138).

11. The prosthetic valve of any one of claims 7 to 10, wherein the clamp further comprises slits (196) extending through the thickness of the side arms.

12. The prosthetic valve of any one of claims 7 to 11, wherein the at least one clamp (180) comprises a first clamp (180a) coupled to the frame at the first locations, and a second clamp (180b) coupled to the frame at the second location, wherein the outer member is coupled to the first clamp, and wherein the inner member is coupled to the second clamp.

13. A method of assembling a prosthetic valve (100), the method comprising:
providing a frame (106) that includes a plurality of inner struts (110b) and outer struts (110a), pivotably coupled to each other at junctions (128) thereof;
coupling at least one clamp (180) to a junction of the frame, wherein the at least one clamp comprises a clamp mid-portion (182), and a couple of opposing resilient side arms (184) continuously extending from the clamp mid-portion;
coupling a component of an expansion and locking assembly (140) to the at least one clamp by sliding the component through the resilient side arms, thereby expanding them away from each other, and allowing the side arms to snap back toward each other once the component is situated within the clamp, wherein the component of the expansion and locking assembly comprises an outer member (142) and/or an inner member (160), configured to expand the prosthetic valve when moved axially toward each other.

14. The method of claim 13, wherein coupling the component of an expansion and locking assembly to the at least one clamp comprises snapping the side arms over a recess (156) of the component.

15. The method of any one of claims 13 or 14, wherein the at least one clamp comprises a first clamp having clamp axial arms extending axially from the side arms, and wherein coupling the component of an expansion and locking assembly to the at least one clamp comprises coupling the outer member to the first clamp, optionally further comprising a step of attaching commissures (138) to the prosthetic valves, by coupling leaflet tabs of each couple of adjacent leaflets (136) of a commissure to the clamp axial arms.

## Patentansprüche

1. Klappenprothese (100), umfassend:
einen Rahmen (106), der zwischen einer radial komprimierten und einer radial expandierten Konfiguration beweglich ist;
zumindest eine Klemme (180), die mit dem Rahmen gekoppelt ist, umfassend: einen Klemmen-Mittelabschnitt (182), der eine Öffnung (190) umfasst, die sich durch dessen Dicke erstreckt, und ein Paar von gegenüberliegenden Seitenarmen (184), die sich kontinuierlich vom Klemmen-Mittelabschnitt erstrecken; und
zumindest einen Expansions- und Verriegelungsmechanismus (140), umfassend:
ein äußeres Element (142), das mit dem Rahmen an einer ersten Position gekoppelt ist; und
ein inneres Element (160), das mit dem Rahmen an einer zweiten Position gekoppelt ist, die von der ersten Position beabstandet ist, wobei sich das innere Element zumindest teilweise in das äußere Element erstreckt;
wobei eine Bewegung des inneren Elements in eine erste Richtung relativ zum äußeren Element bewirkt, dass sich der Rahmen axial verkürzt und radial expandiert;
wobei die Seitenarme elastisch voneinander weg expandierbar sind und in Abwesenheit einer darauf ausgeübten Expansionskraft nach innen zueinander gerichtet sind; und
wobei zumindest eines von: dem äußeren Element und/oder dem inneren Element, mit dem Rahmen über die zumindest eine Klemme gekoppelt ist.

2. Klappenprothese gemäß Anspruch 1, wobei der Rahmen eine Vielzahl von inneren Streben (110b) und eine Vielzahl von äußeren Streben (110a) umfasst, die an Verbindungsstellen (128) schwenkbar miteinander verbunden sind, und wobei die zumindest eine Klemme an einer inneren Strebe und an einer äußeren Strebe an einer dazwischen gebildeten Verbindungsstelle befestigt ist.

3. Klappenprothese gemäß einem der Ansprüche 1 oder 2, wobei jeder der Seitenarme einen versetzten Abschnitt (186), der sich kontinuierlich vom Klemmen-Mittelabschnitt erstreckt, und einen bogenförmigen Abschnitt (188) umfasst, der sich kontinuierlich vom versetzten Abschnitt erstreckt.

4. Klappenprothese gemäß einem der Ansprüche 1 bis 3, wobei die zumindest eine Klemme ferner Klemmen-Axialarme (194) umfasst, die sich axial von den Seitenarmen erstrecken; optional:
a.) wobei die Klemmen-Axialarme radial nach innen in Bezug auf das äußere Element versetzt sind, und/oder
b.) ferner umfassend eine Vielzahl von Klappensegeln (136), wobei jedes Klappensegel ein Paar von gegensätzlich ausgerichteten Laschen (137) umfasst, wobei die Laschen von jeweils zwei benachbarten Klappensegeln mit den Klemmen-Axialarmen gekoppelt sind und dadurch eine Kommissur (138) bilden.

5. Klappenprothese gemäß einem der vorhergehenden Ansprüche, wobei die Klemme ferner Schlitze (196) umfasst, die sich durch die Dicke der Seitenarme erstrecken.

6. Klappenprothese gemäß einem der vorhergehenden Ansprüche, wobei die zumindest eine Klemme (180) eine erste Klemme (180a), die mit dem Rahmen an der ersten Position gekoppelt ist, und eine zweite Klemme (180b) umfasst, die mit dem Rahmen an der zweiten Position gekoppelt ist, wobei das äußere Element mit der ersten Klemme gekoppelt ist, und wobei das innere Element mit der zweiten Klemme gekoppelt ist.

7. Klappenprothese (100), umfassend:
einen Rahmen (106), der zwischen einer radial komprimierten und einer radial expandierten Konfiguration beweglich ist;
zumindest eine Klemme (180), die mit dem Rahmen gekoppelt ist, umfassend: einen Klemmen-Mittelabschnitt (182), der einen Klemmen-Befestigungsfortsatz (192) umfasst, der sich radial nach außen davon erstreckt, und ein Paar von gegenüberliegenden Seitenarmen (184), die sich kontinuierlich von dem Klemmen-Mittelabschnitt erstrecken; und
zumindest einen Expansions- und Verriegelungsmechanismus (140), umfassend:
ein äußeres Element (142), das mit dem Rahmen an einer ersten Position gekoppelt ist; und
ein inneres Element (160), das mit dem Rahmen an einer zweiten Position gekoppelt ist, die von der ersten Position beabstandet ist, wobei sich das innere Element zumindest teilweise in das äußere Element erstreckt;
wobei eine Bewegung des inneren Elements in eine erste Richtung relativ zum äußeren Element bewirkt, dass sich der Rahmen axial verkürzt und radial expandiert;
wobei die Seitenarme elastisch voneinander weg expandierbar sind und in Abwesenheit einer darauf ausgeübten Expansionskraft nach innen zueinander gerichtet sind; und
wobei zumindest eines von: dem äußeren Element und/oder dem inneren Element, mit dem Rahmen über die zumindest eine Klemme gekoppelt ist.

8. Klappenprothese gemäß Anspruch 7, wobei der Rahmen eine Vielzahl von inneren Streben (110b) und eine Vielzahl von äußeren Streben (110a) umfasst, die an Verbindungsstellen (128) schwenkbar miteinander verbunden sind, und wobei die zumindest eine Klemme an einer inneren Strebe und einer äußeren Strebe an einer dazwischen gebildeten Verbindungsstelle befestigt ist.

9. Klappenprothese gemäß einem der Ansprüche 7 oder 8, wobei jeder der Seitenarme einen versetzten Abschnitt (186), der sich kontinuierlich vom Klemmen-Mittelabschnitt erstreckt, und einen bogenförmigen Abschnitt (188) umfasst, der sich kontinuierlich vom versetzten Abschnitt erstreckt.

10. Klappenprothese gemäß einem der Ansprüche 7 bis 9, wobei die zumindest eine Klemme ferner Klemmen-Axialarme (194) umfasst, die sich axial von den Seitenarmen erstrecken; optional:
a.) wobei die Klemmen-Axialarme radial nach innen in Bezug auf das äußere Element versetzt sind, und/oder
b.) ferner umfassend eine Vielzahl von Klappensegeln (136), wobei jedes Klappensegel ein Paar von gegensätzlich ausgerichteten Laschen (137) umfasst, wobei die Laschen von jeweils zwei benachbarten Klappensegeln mit den Klemmen-Axialarmen gekoppelt sind und dadurch eine Kommissur (138) bilden.

11. Klappenprothese gemäß einem der Ansprüche 7 bis 10, wobei die Klemme ferner Schlitze (196) umfasst, die sich durch die Dicke der Seitenarme erstrecken.

12. Klappenprothese gemäß einem der Ansprüche 7 bis 11, wobei die zumindest eine Klemme (180) eine erste Klemme (180a), die mit dem Rahmen an den ersten Positionen gekoppelt ist, und eine zweite Klemme (180b) umfasst, die mit dem Rahmen an der zweiten Position gekoppelt ist, wobei das äußere Element mit der ersten Klemme gekoppelt ist, und wobei das innere Element mit der zweiten Klemme gekoppelt ist.

13. Verfahren zum Zusammenbauen einer Klappenprothese (100), wobei das Verfahren umfasst:
Bereitstellen eines Rahmens (106), der eine Vielzahl von inneren Streben (110b) und äußeren Streben (110a) aufweist, die an deren Verbindungsstellen (128) schwenkbar miteinander gekoppelt sind;
Koppeln zumindest einer Klemme (180) mit einer Verbindungsstelle des Rahmens, wobei die zumindest eine Klemme einen Klemmen-Mittelabschnitt (182) und ein Paar von gegenüberliegenden elastischen Seitenarmen (184) umfasst, die sich kontinuierlich vom Klemmen-Mittelabschnitt erstrecken;
Koppeln einer Komponente einer Expansions- und Verriegelungsanordnung (140) mit der zumindest einen Klemme durch Schieben der Komponente durch die elastischen Seitenarme, wodurch sie diese voneinander weg expandiert, und Ermöglichen, dass die Seitenarme zueinander zurückschnappen, sobald die Komponente innerhalb der Klemme angeordnet ist, wobei die Komponente der Expansions- und Verriegelungsanordnung ein äußeres Element (142) und/oder ein inneres Element (160) umfasst, das/die dazu ausgelegt ist/sind, die Klappenprothese zu expandieren, wenn sie axial aufeinander zu bewegt werden.

14. Verfahren gemäß Anspruch 13, wobei das Koppeln der Komponente einer Expansions- und Verriegelungsanordnung mit der zumindest einen Klemme ein Einrasten der Seitenarme über eine Aussparung (156) der Komponente umfasst.

15. Verfahren gemäß einem der Ansprüche 13 oder 14, wobei die zumindest eine Klemme eine erste Klemme mit Klemmen-Axialarmen umfasst, die sich axial von den Seitenarmen erstrecken, und wobei das Koppeln der Komponente einer Expansions- und Verriegelungsanordnung mit der zumindest einen Klemme das Koppeln des äußeren Elements mit der ersten Klemme umfasst, optional ferner umfassend einen Schritt des Befestigens von Kommissuren (138) an den Klappenprothesen, durch Koppeln von Klappensegel-Laschen jeweils zweier benachbarter Klappensegel (136) einer Kommissur mit den Klemmen-Axialarmen.

## Revendications

1. Valvule prothétique (100), comprenant :
un cadre (106) mobile entre une configuration radialement comprimée et une configuration radialement déployée ;
au moins une pince (180) accouplée au cadre, comprenant : une partie médiane (182) de pince comprenant une ouverture (190) s'étendant à travers son épaisseur et une paire de bras latéraux (184) opposés s'étendant en continu à partir de la partie médiane de pince ; et
au moins un mécanisme de déploiement et de verrouillage (140), comprenant :
un élément externe (142), accouplé au cadre à un premier emplacement ; et
un élément interne (160), accouplé au cadre à un second emplacement écarté du premier emplacement, l'élément interne s'étendant au moins partiellement dans l'élément externe ;
le déplacement de l'élément interne dans un premier sens, par rapport à l'élément externe, amenant le cadre à se raccourcir axialement et à se déployer radialement ;
les bras latéraux étant élastiquement extensibles à l'opposé l'un de l'autre et étant sollicités vers l'intérieur l'un vers l'autre en l'absence d'une force de déploiement appliquée à ceux-ci ; et
au moins un élément parmi : l'élément externe et/ou l'élément interne étant accouplé au cadre par l'intermédiaire de ladite au moins une pince.

2. Valvule prothétique selon la revendication 1, le cadre comprenant une pluralité d'entretoises internes (110b) et une pluralité d'entretoises externes (110a), interconnectées de manière pivotante au niveau de jonctions (128) et ladite au moins une pince étant fixée à une entretoise interne et à une entretoise externe au niveau d'une jonction formée entre celles-ci.

3. Valvule prothétique selon l'une quelconque des revendications 1 ou 2, chacun des bras latéraux comprenant une partie de décalage (186) s'étendant en continu à partir de la partie médiane de pince et une partie arquée (188) s'étendant en continu à partir de la partie de décalage.

4. Valvule prothétique selon l'une quelconque des revendications 1 à 3, ladite au moins une pince comprenant en outre des bras axiaux (194) de pince, s'étendant axialement à partir des bras latéraux ; éventuellement :
a.) les bras axiaux de pince étant décalés radialement vers l'intérieur par rapport à l'élément externe et/ou
b.) comprenant en outre une pluralité de feuillets (136), chaque feuillet comprenant une paire de languettes (137) dirigées de manière opposée, les languettes de chaque paire de feuillets adjacents étant accouplées aux bras axiaux de pince, formant ainsi une commissure (138).

5. Valvule prothétique selon l'une quelconque revendication précédente, la pince comprenant en outre des fentes (196) s'étendant à travers l'épaisseur des bras latéraux.

6. Valvule prothétique selon l'une quelconque revendication précédente, ladite au moins une pince (180) comprenant une première pince (180a) accouplée au cadre au niveau du premier emplacement et une seconde pince (180b) accouplée au cadre au niveau du second emplacement, l'élément externe étant accouplé à la première pince et l'élément interne étant couplé à la seconde pince.

7. Valvule prothétique (100) comprenant :
un cadre (106) mobile entre une configuration radialement comprimée et une configuration radialement déployée ;
au moins une pince (180) accouplée au cadre, comprenant : une partie médiane (182) de pince comprenant une extension de fixation (192) de pince s'étendant radialement vers l'extérieur à partir de celle-ci et une paire de bras latéraux (184) opposés s'étendant en continu à partir de la partie médiane de pince ; et
au moins un mécanisme de déploiement et de verrouillage (140), comprenant :
un élément externe (142), accouplé au cadre à un premier emplacement ; et
un élément interne (160), accouplé au cadre à un second emplacement écarté du premier emplacement, l'élément interne s'étendant au moins partiellement dans l'élément externe ;
le déplacement de l'élément interne dans un premier sens, par rapport à l'élément externe, amenant le cadre à se raccourcir axialement et à se déployer radialement ;
les bras latéraux étant élastiquement extensibles à l'opposé l'un de l'autre et étant sollicités vers l'intérieur l'un vers l'autre en l'absence d'une force de déploiement appliquée à ceux-ci ; et
au moins un élément parmi : l'élément externe et/ou l'élément interne étant accouplé au cadre par l'intermédiaire de ladite au moins une pince.

8. Valvule prothétique selon la revendication 7, le cadre comprenant une pluralité d'entretoises internes (110b) et une pluralité d'entretoises externes (110a), interconnectées de manière pivotante au niveau de jonctions (128) et ladite au moins une pince étant fixée à une entretoise interne et à une entretoise externe au niveau d'une jonction formée entre celles-ci.

9. Valvule prothétique selon l'une quelconque des revendications 7 ou 8, chacun des bras latéraux comprenant une partie de décalage (186) s'étendant en continu à partir de la partie médiane de pince et une partie arquée (188) s'étendant en continu à partir de la partie de décalage.

10. Valvule prothétique selon l'une quelconque des revendications 7 à 9, ladite au moins une pince comprenant en outre des bras axiaux (194) de pince, s'étendant axialement à partir des bras latéraux ; éventuellement :
a.) les bras axiaux de pince étant décalés radialement vers l'intérieur par rapport à l'élément externe et/ou
b.) comprenant en outre une pluralité de feuillets (136), chaque feuillet comprenant une paire de languettes (137) dirigées de manière opposée, les languettes de chaque paire de feuillets adjacents étant accouplées aux bras axiaux de pince, formant ainsi une commissure (138).

11. Valvule prothétique selon l'une quelconque des revendications 7 à 10, la pince comprenant en outre des fentes (196) s'étendant à travers l'épaisseur des bras latéraux.

12. Valvule prothétique selon l'une quelconque des revendications 7 à 11, ladite au moins une pince (180) comprenant une première pince (180a) accouplée au cadre au niveau des premiers emplacements et une seconde pince (180b) accouplée au cadre au niveau du second emplacement, l'élément externe étant accouplé à la première pince et l'élément interne étant accouplé à la seconde pince.

13. Procédé d'assemblage d'une valvule prothétique (100), le procédé comprenant :
la fourniture d'un cadre (106) qui comprend une pluralité d'entretoises internes (110b) et une pluralité d'entretoises externes (110a), accouplées de manière pivotante les unes aux autres au niveau de jonctions (128) correspondantes ;
l'accouplement de ladite au moins une pince (180) à une jonction du cadre, ladite au moins une pince comprenant une partie médiane (182) de pince et une paire de bras latéraux élastiques (184) opposés s'étendant en continu à partir de la partie médiane de pince ;
l'accouplement d'un composant d'un ensemble de déploiement et de verrouillage (140) à ladite une pince par coulissement du composant à travers les bras latéraux élastiques, ce qui les étend à l'opposé l'un de l'autre et ce qui permet aux bras latéraux de revenir brusquement l'un vers l'autre une fois que le composant est situé à l'intérieur de la pince, le composant de l'ensemble de déploiement et de verrouillage comprenant un élément externe (142) et/ou un élément interne (160), conçus pour déployer la valvule prothétique lorsqu'ils sont déplacés axialement l'un vers l'autre.

14. Procédé selon la revendication 13, l'accouplement du composant d'un ensemble de déploiement et de verrouillage à ladite au moins une pince comprenant l'enclenchement des bras latéraux par-dessus d'un renfoncement (156) du composant.

15. Procédé selon l'une quelconque des revendications 13 ou 14, ladite au moins une pince comprenant une première pince présentant des bras axiaux de pince s'étendant axialement à partir des bras latéraux et l'accouplement du composant d'un ensemble de déploiement et de verrouillage à ladite au moins une pince comprenant l'accouplement de l'élément externe à la première pince, comprenant éventuellement en outre une étape de fixation de commissures (138) aux valvules prothétiques, par accouplement de languettes de feuillet de chaque paire de feuillets adjacents (136) d'une commissure aux bras axiaux de pince.
